# EUROPEAN PATENT APPLICATION

(11) **EP 2 256 677 A1**
(43) Date of publication of application: **01.12.2010**
(21) Application number: 08711854.3
(22) Date of filing: 22.02.2008
(51) Int. Cl.: G06Q 10/00

(54) **WORKFLOW PROCESSING PROGRAM, METHOD, AND DEVICE**

(71) Applicant: Fujitsu Limited, Kawasaki-shi, Kanagawa 211-8588 (JP)
(72) Inventor: KAWAMURA, Akira, Kawasaki-shi Kanagawa 211-8588 (JP); HARA, Hirotaka, Kawasaki-shi Kanagawa 211-8588 (JP)
(74) Representative: Stebbing, Timothy Charles
(86) International application number: PCT/JP2008/053086
(87) International publication number: WO 2009/104276

(57) **Abstract**

In order to make it easy to grasp a feature of the entire business flow carried out by a user by enabling to appropriately carry out classification of the business flows, this business flow processing method includes extracting data of a series of works carried out for each case from a database storing results of the works, generating process instances in which work names of the works carried out are arranged in time series; judging, for each of the process instances, whether or not a backtracking from a first work to a second work carried out prior to the first work occurs; deleting, for each type of backtracking patterns, a repeated backtracking from the process instance in which the backtracking occurs; counting, for each type, the number of process instances after deletion of the repeated backtracking; and identifying, based on counting results, a process instance after the deletion of the repeated backtracking, whose appearance frequency is equal to or greater than a predetermined reference, and outputting the identified process instance as a main business flow.

## Description

### Technical Field

This invention relates to an information processing technique for a business process analysis.

### Background Technology

For Business Process Re-engineering (BPR), it is necessary to analyze currently operated business systems in the company. For such a purpose, a technique disclosed, for example, in Japanese laid-open patent publication No. 2005-115494 is used. In this publication, following matters are disclosed.

Namely, (1) event data, which is information representing execution states of respective applications allocated in different business systems, is collected according to methods corresponding to the respective applications and is queued into an event queue. Incidentally, in this publication, the event indicates a certain business process was executed in the business systems, and is data including a start time and end time of the business process and associated attributes. The event data is extracted according to event extraction definition allocated in the respective business systems by an application for the event data extraction for each business system. In each of the business systems, the extracted event data is converted into a common extensible Markup Language (XML) format to queue the converted data into an event queue of an event management apparatus managing the event data. For example, Java (registered trademark) Message Service (JMS) is utilized for this queuing.

(2) In the event management apparatus, the event data queued in the event queue is aggregated for each unit of the business data and stored in an event management database (DB) after associating the business data units. In this publication, the business data means data shared between business processes in a certain collected unit. (3) Narrowing the business data is carried out based on inputted retrieval condition (e.g. event occurrence period, associated attributes and the like.). (4) Data associated with the narrowed business data is expanded and displayed by a tree, and the process from arbitrary data is tracked. (5) An event associated with the business data expanded by the tree is retrieved, and the business associated with this event is depicted by a tracking view to display the execution state of the current business flow. In this publication, the tracking is a method for confirming which business process is executed or which business process is not executed in the business processes that correspond to an entire business flow executed across the predefined business systems.

It is necessary for such a technique described in this publication to introduce the applications for the event data extraction for each business system, and the business systems have to be modified or loads unnecessary for the business process execution is provided.

In addition, in such a publication, a configuration is not disclosed that the execution frequency of the business flows is analyzed to categorize them into standard business flow and exceptional business flows, and any problems in this categorization are not suggested or disclosed.
Patent Document 1:Japanese Laid-open Patent Publication 2005-115494

### Summary of the Invention

### Problem to be solved by this Invention

Therefore, an object of this invention is to provide a technique enabling a user to easily grasp a feature of the entire executed business flow by appropriately carrying out the categorization of the business flows.

### Means for solving the Problem

A business flow processing method according to this invention includes: extracting data of a series of works carried out for each case from a database storing results of the works, generating and storing into a process instance data storage device, process instances in which work names of the works carried out are arranged in time series; judging, for each of the process instances stored in the process instance data storage device, whether or not a backtracking from a first work to a second work carried out prior to the first work occurs; deleting, for each type of backtracking patterns, a repeated backtracking (i.e. a backtracking making it difficult to grasp the entire image of the business) from the process instance in which the backtracking occurs, and storing a process instance after deletion of the repeated backtracking into a simplified process instance data storage device; counting, for each type, the number or process instances stored in the simplified process instance data storage device; and identifying, based on counting results, a process instance whose appearance frequency is equal to or greater than a predetermined reference and which is stored in the simplified process instance data storage device, and outputting the identified process instance as a main business flow.

By doing so, even if the same backtrackings occurred a let of times, it is possible to unify them into one backtracking, and to easily identify the main business flow, which is important on grasping the feature of the entire business flow.

Incidentally, the aforementioned outputting may include superimposing the identified process instances. This purpose is to make the user grasp the main business flow more easily.

Furthermore, the aforementioned outputting may include outputting, as an exceptional flow, the process instances other than identified process instances. This purpose is to grasp occurrence states of the exceptional flows in order to improve the business.

Furthermore, this invention may further include: judging, for each process instance stored in the process instance data storage device, whether or not an iteration from a third work in the process instance to the third work occurs; and deleting, for each type of iteration patterns, an additional iteration (i.e. iteration making it difficult to grasp the entire image of the business) from the process instance that the iteration occurs, and storing the process instance after the deletion of the additional iteration into the process instance data storage device. By doing so, even if the same iterations occur a lot of times, it is possible to unify them into one iteration, and to identify the main business flow, which is important on grasping the feature of the entire business flow.

Furthermore, this invention may further include: judging, for each process instance stored in the simplified process instance data storage device, whether or not an iteration from a third work in the process instance to the third work occurs; and deleting, for each type of iteration patterns, an additional iteration (i.e. iteration making it difficult to grasp the entire image of the business) from the process instance that the iteration occurs, and storing the process instance after the deletion of the additional iteration into the simplified process instance data storage device. The deletion of the repeated iteration may be carried out after or before the repeated backtracking. In addition, the deletion of the additional backtracking or the deletion of the additional iteration may be carried out independently.

Incidentally, it is possible to create a program causing a computer to execute the methods according to the invention, and such a program is stored in a computer readable storage medium or storage device such as a flexible disk, CD-ROM, magneto-optic disk, a semiconductor memory, and hard disk. In addition, the program may be distributed through a network by digital signals. Incidentally, the intermediate processing result is temporarily stored in a storage device such as a main memory or the like.

### Brief description of the drawings

Fig. 1 is a functional block diagram in an embodiment of this invention;
Figs. 2A to 2D are diagrams to explain an outline of this embodiment of this invention;
Fig. 3 is a diagram depicting a main processing flow in the embodiment of this invention;
Fig. 4A is a diagram depicting schema information of an order DB, which is an example of extraction data, and Fig. 4B is a diagram depicting records in the order DB;
Fig. 5A is a diagram depicting schema information of a production DB, which is an example of extraction data, and Fig. 5B is a diagram depicting records in the production DB;
Fig. 6A is a diagram depicting schema information of an arrangement DB, which is an example of extraction data, and Fig. 6B is a diagram depicting records in the arrangement DB;
Fig. 7A is a diagram depicting schema information of a delivery DB, which is an example of extraction data, and Fig. 7B is a diagram depicting records in the delivery DB;
Fig. 8A is a diagram depicting schema information of a product number DB, which is an example of extraction data, and Fig. 8B is a diagram depicting records in the product number DB;
Fig. 9A is a diagram depicting a data example of the order DB in a CSV format, and Fig. 9B is a diagram depicting an example that the data of the order DB is tabulated;
Fig. 10A is a diagram depicting a data example of the production DB in a CSV format, and Fig. 10B is a diagram depicting an example that the data of the production DB is tabulated;
Fig. 12A is a diagram depicting a data example of the arrangement DB in a CSV format, and Fig. 11B is a diagram depicting an example that the data of the arrangement DB is tabulated;

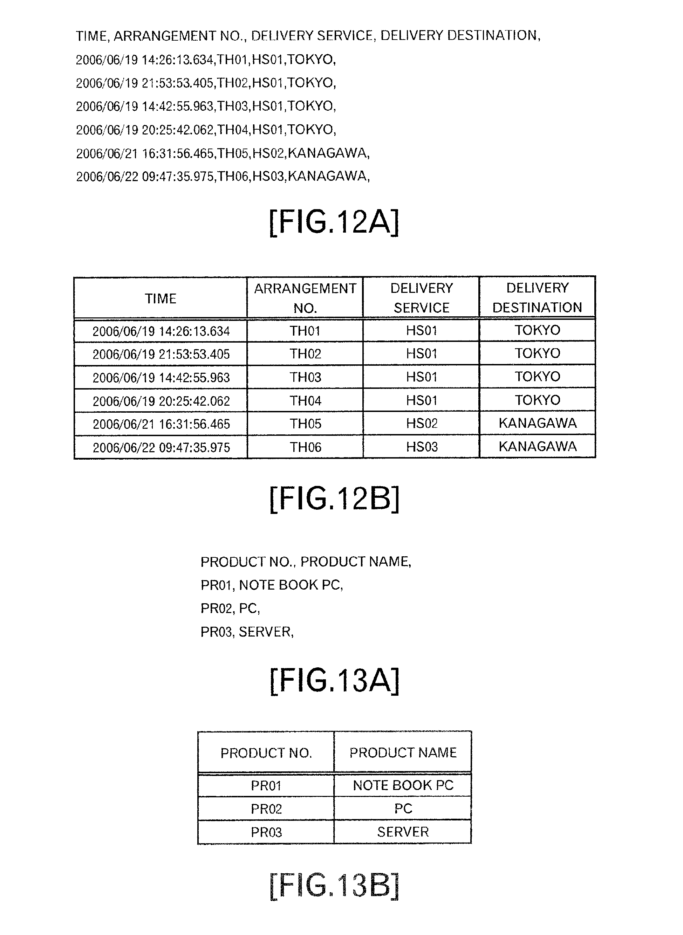

Fig. 12A is a diagram depicting a data example of the delivery DB in a CSV format, and Fig. 12B is a diagram depicting an example that the data of the delivery DB is tabulated;
Fig. 13A is a diagram depicting a data example of the product number DB in a CSV format, and Fig. 13B is a diagram depicting an example that the data of the product number DB is tabulated;

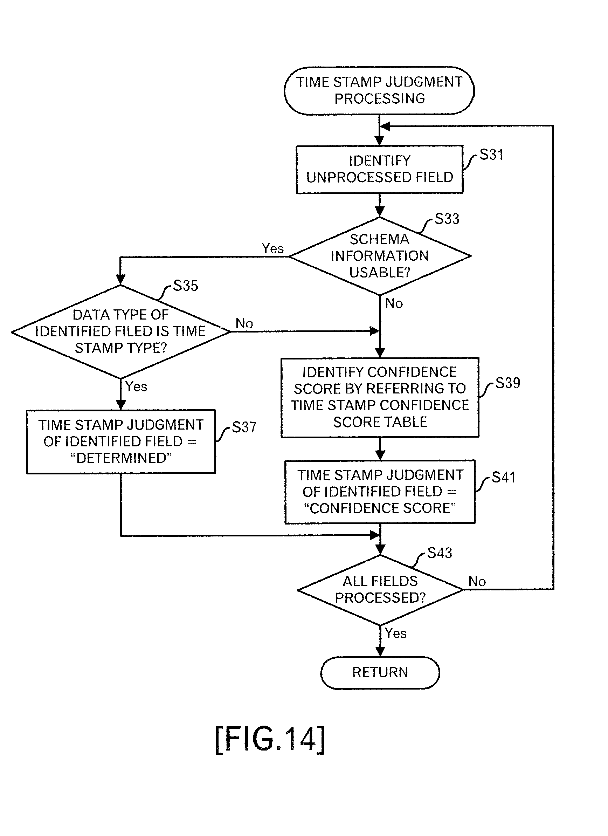

Fig. 14 is a diagram depicting a processing flow of a time stamp judgment processing;

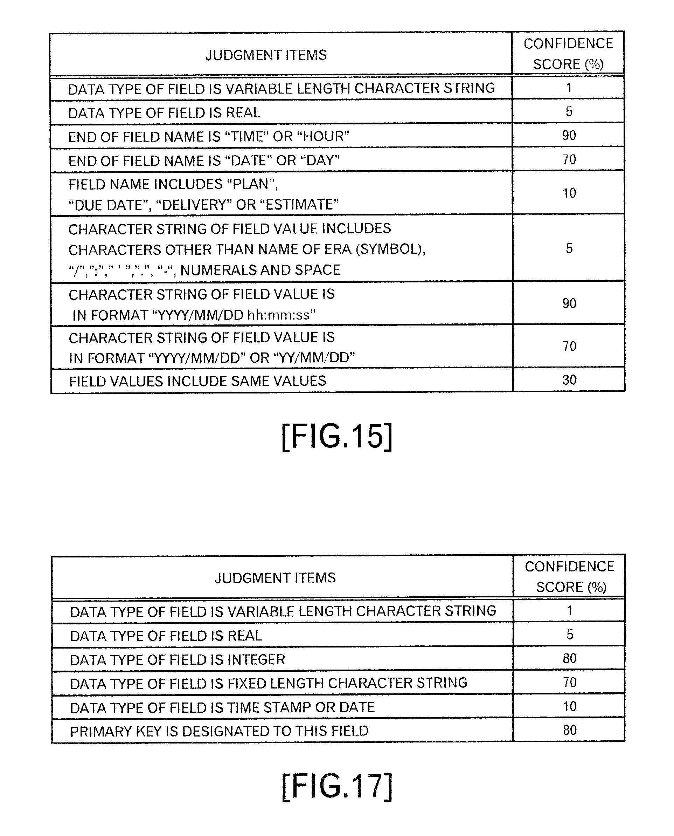

Fig. 15 is a diagram depicting an example of a time stamp confidence score table;

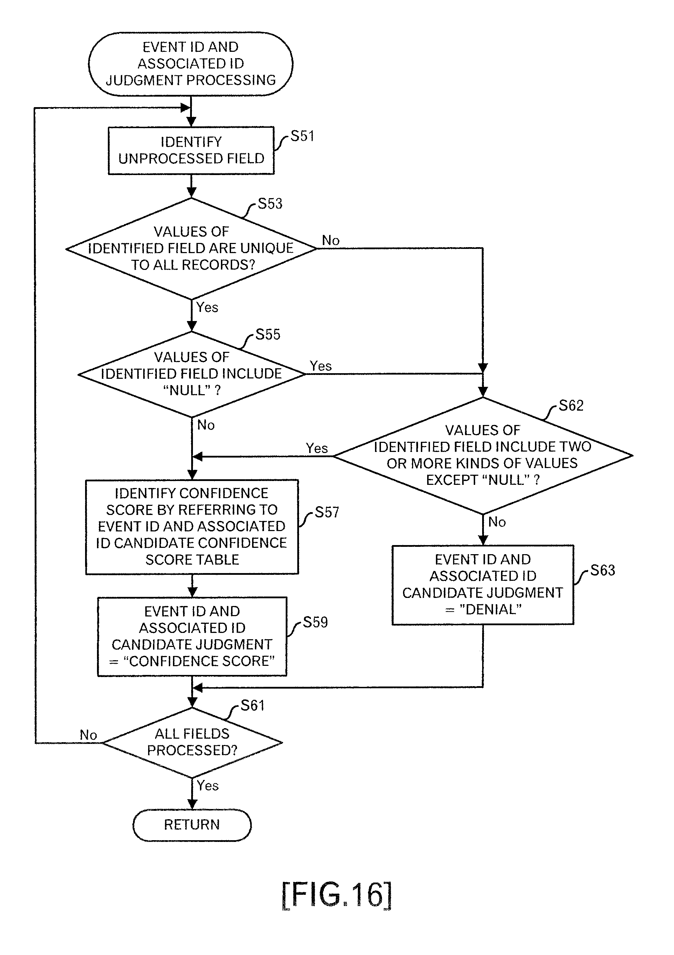

Fig. 16 is a diagram depicting a processing flow of an event ID and associated ID candidate judgment processing;
Fig. 17 is a diagram depicting an example of an event ID and associated ID candidate confidence score table;

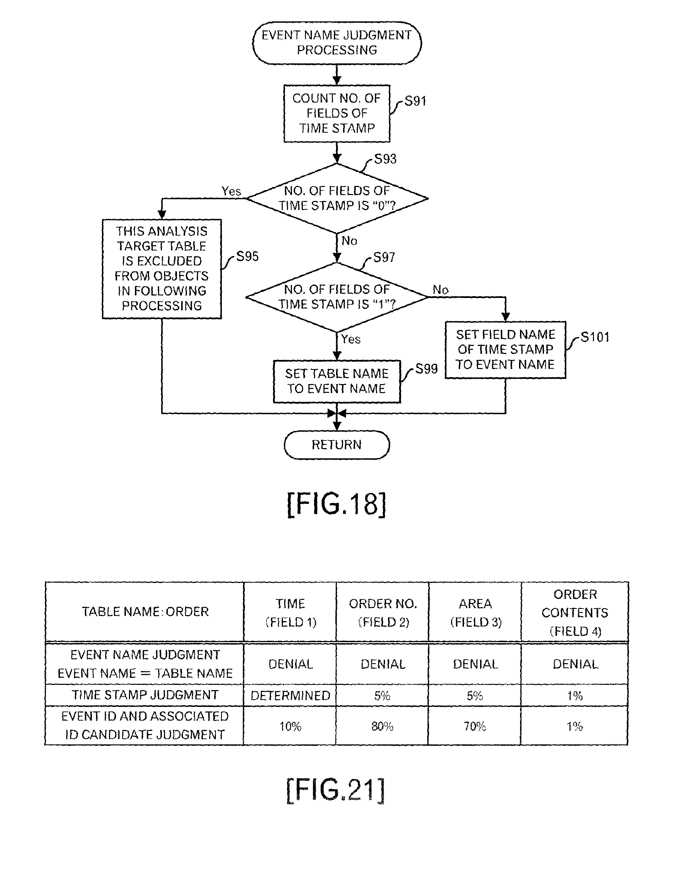

Fig. 18 is a diagram depicting a processing flow of an event name judgment processing;

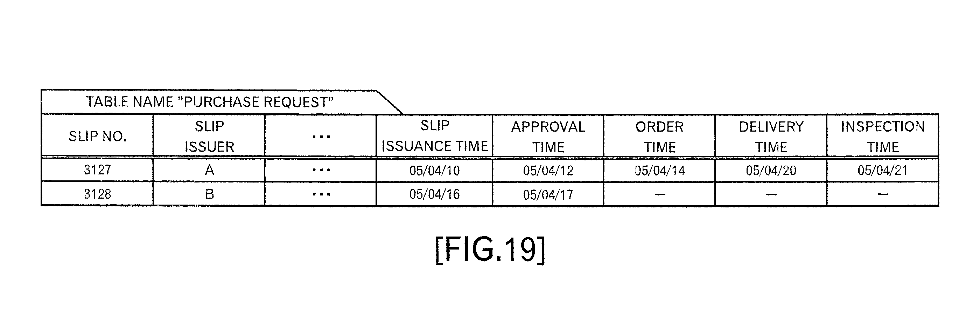

Fig. 19 is a diagram depicting an example of a table including plural time stamps;

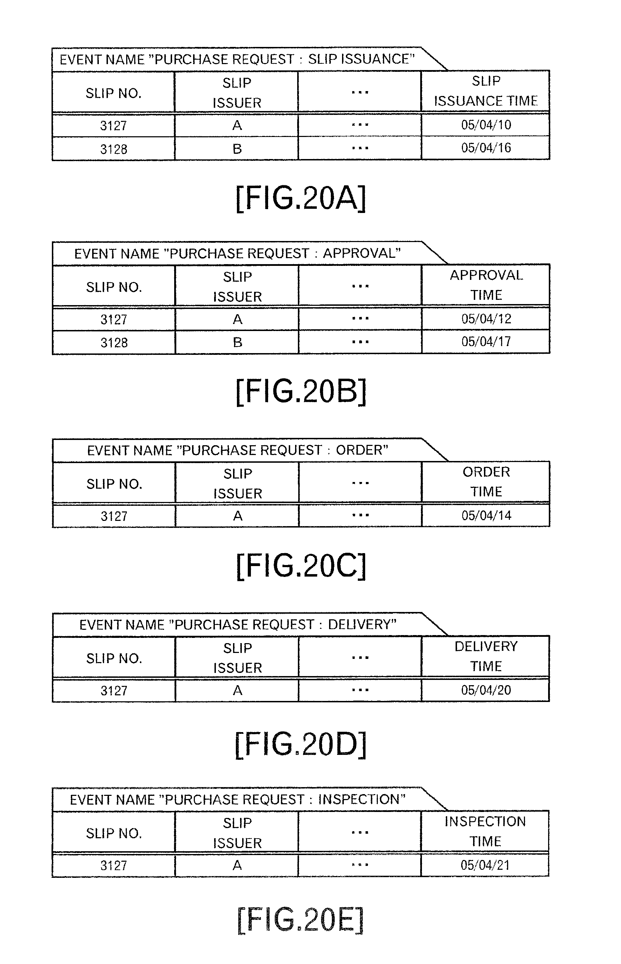

Figs 20A to 20E are diagrams depicting an example of dividing the table of Fig. 19 into plural tables for each event;
Fig. 21 is a diagram depicting an example of judgment display for each element of event candidate data of the order DB in case that the schema information exists;

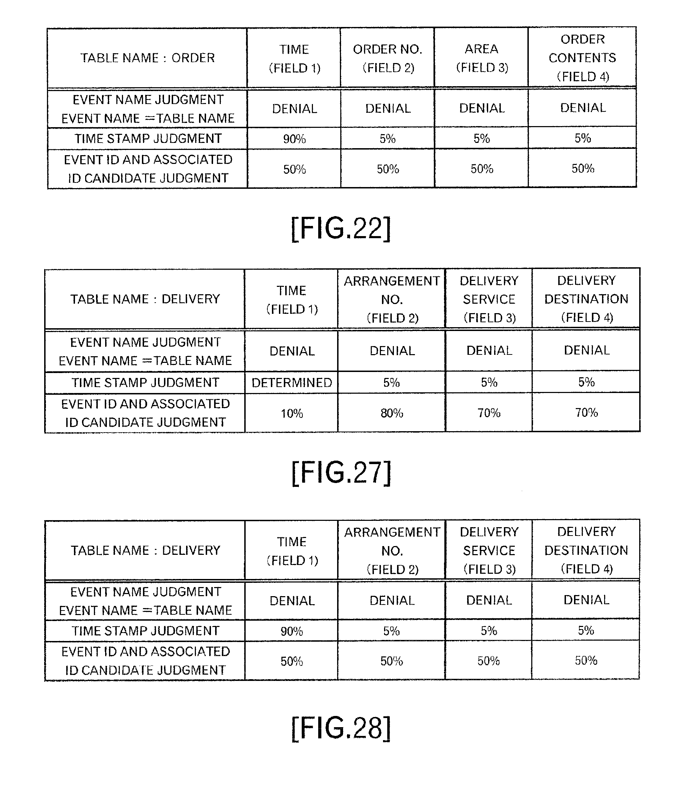

Fig. 22 is a diagram depicting an example of judgment display for each element of event candidate of the order DB in case of data in a CSV format;

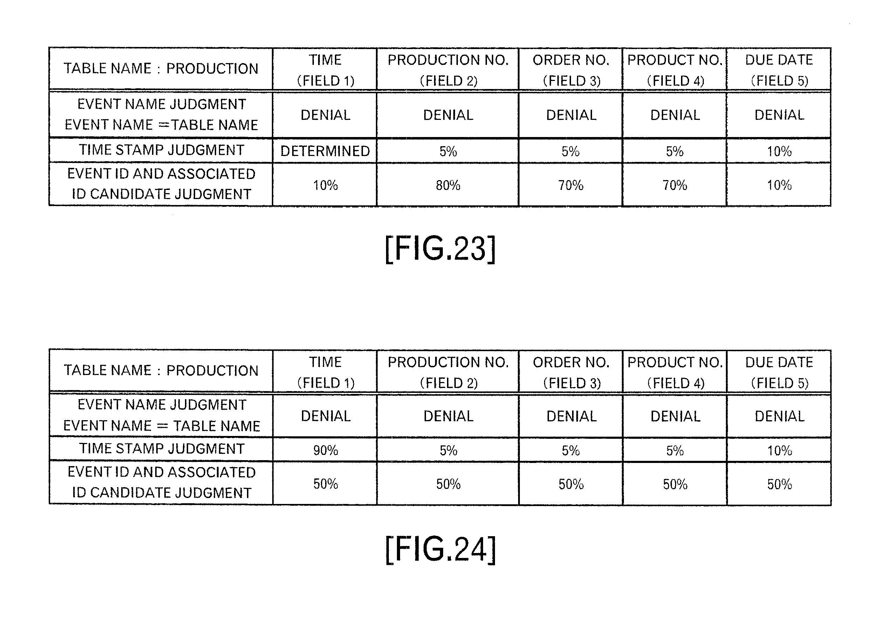

Fig. 23 is a diagram depicting an example of judgment display for each element of event candidate of the production DB in case that the schema information exists;
Fig. 24 is a diagram depicting an example of judgment display for each element of event candidate of the production DB in case of data in the CSV format;

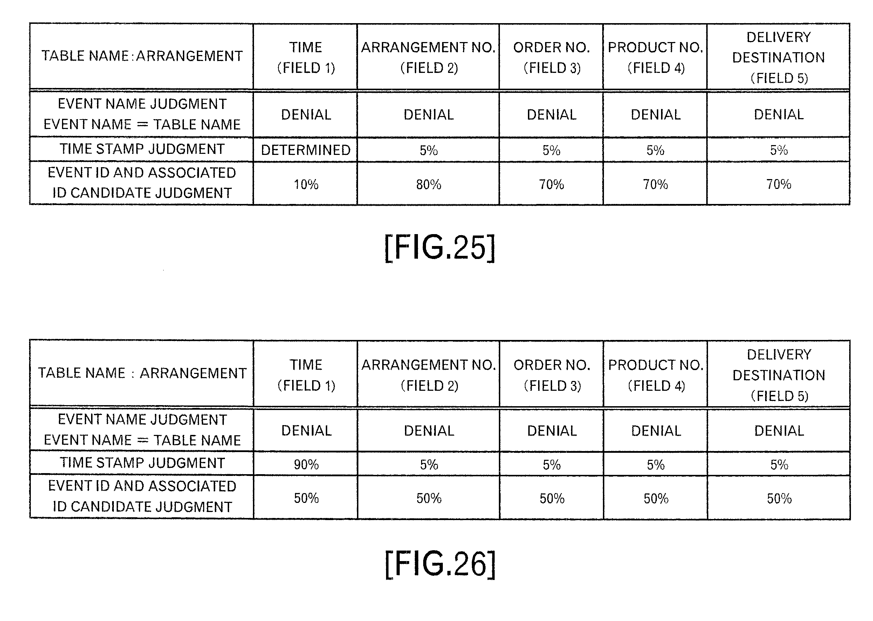

Fig. 25 is a diagram depicting an example of judgment display for each element of event candidate of the arrangement DB in case that the schema information exists;
Fig. 26 is a diagram depicting an example of judgment display for each element of event candidate data of the arrangement DB in case of data in the CSV format;
Fig. 27 is a diagram depicting an example of judgment display for each element of event candidate of the delivery DB in case that the schema information exists;
Fig. 28 is a diagram depicting an example of judgment display for each element of event candidate data of the delivery DB in case of data in the CSV format;

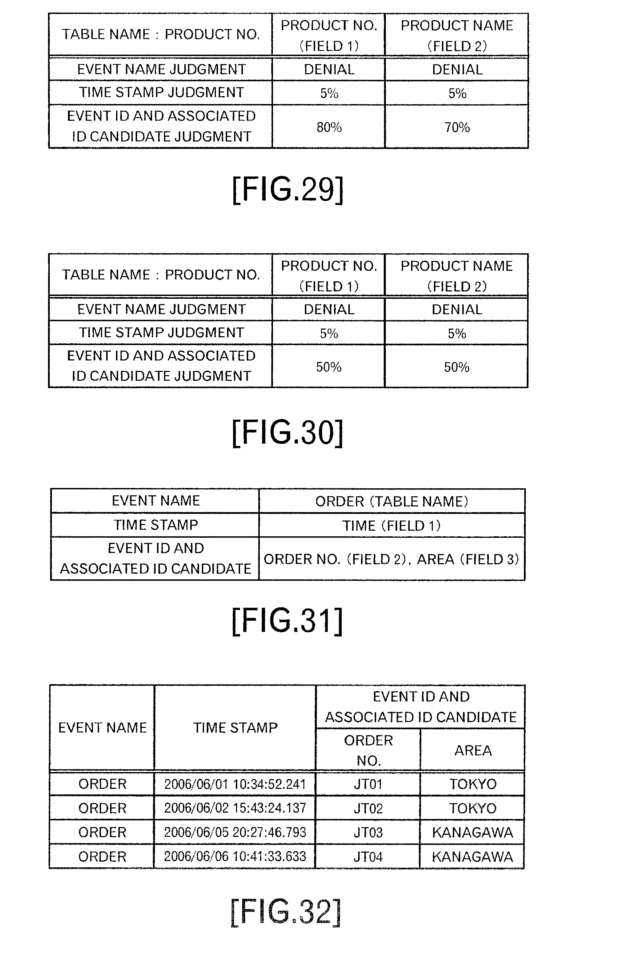

Fig. 29 is a diagram depicting an example of judgment display for each element of event candidate data of the product number DB in case that the schema information exists;
Fig. 30 is a diagram depicting an example of judgment display for each element of event candidate of the product number DB in case of data in the CSV format;
Fig. 31 is a diagram depicting an example of selection results for the respective elements of the event candidate data;
Fig. 32 is a diagram depicting an example of the event candidate data generated from data of the order DB in case that the schema information exists;

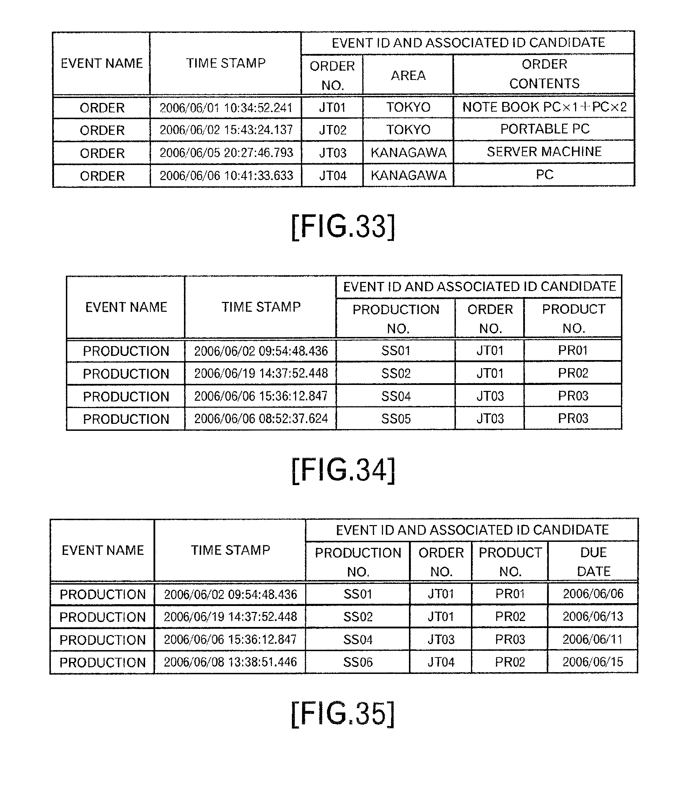

Fig. 33 is a diagram depicting an example of the event candidate data generated from data of the order DB in case of the data in the CSV format;
Fig. 34 is a diagram depicting an example of the event candidate data generated from data of the production DB in case that the schema information exists;
Fig. 35 is a diagram depicting an example of the event candidate data generated from data of the production DB in case of the data in the CSV format;

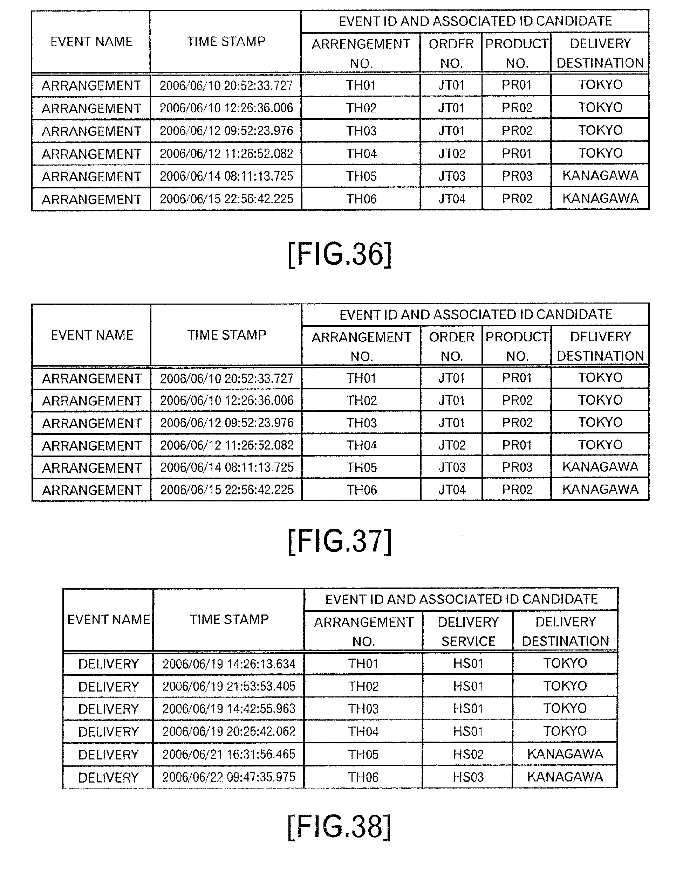

Fig. 36 is a diagram depicting an example of the event candidate data generated from data of the arrangement DB in case that the schema information exists;
Fig. 37 is a diagram depicting an example of the event candidate data generated from data of the arrangement DB in case of the data in the CSV format;
Fig. 38 is a diagram depicting an example of the event candidate data generated from data of the delivery DB in case that the schema information exists;

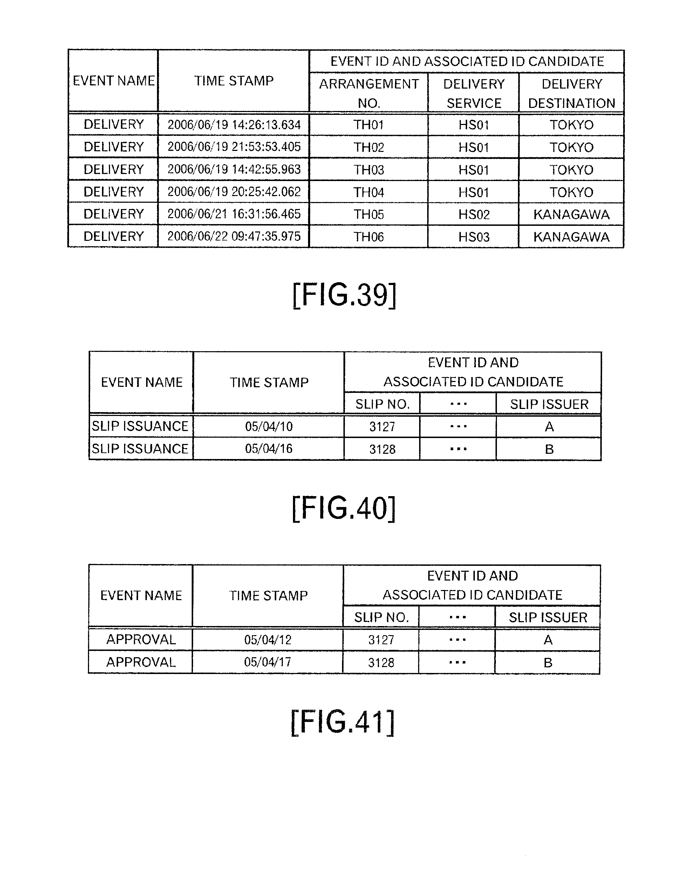

Fig. 39 is a diagram depicting an example of the event candidate data generated from data of the delivery DB in case of the data in the CSV format;
Fig. 40 is a diagram depicting an example of the event candidate data concerning slip issuance of Fig. 19;
Fig. 41 is a diagram depicting an example of the event candidate data concerning approval of Fig. 19;

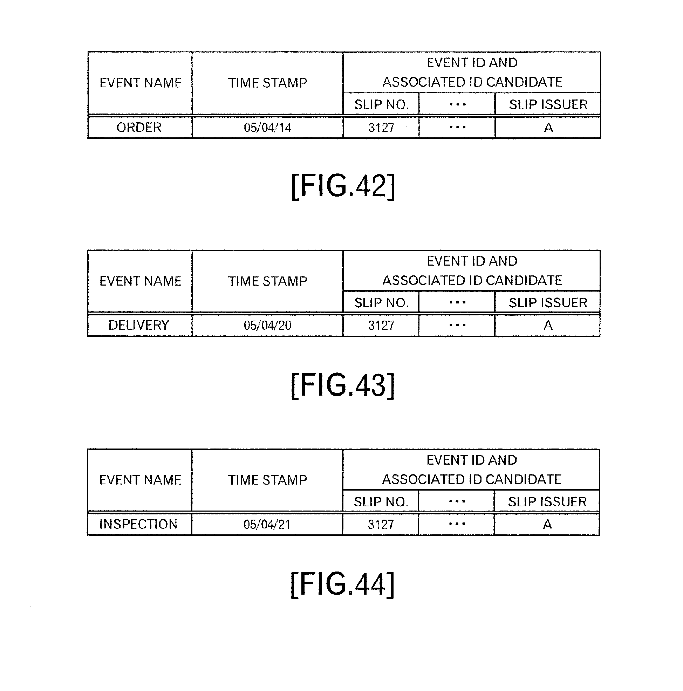

Fig. 42 is a diagram depicting an example of the event candidate data concerning the order of Fig. 19;
Fig. 43 is a diagram depicting an example of the event candidate data concerning delivery of Fig. 19;
Fig. 44 is a diagram depicting an example of the event candidate data concerning inspection of Fig. 19;

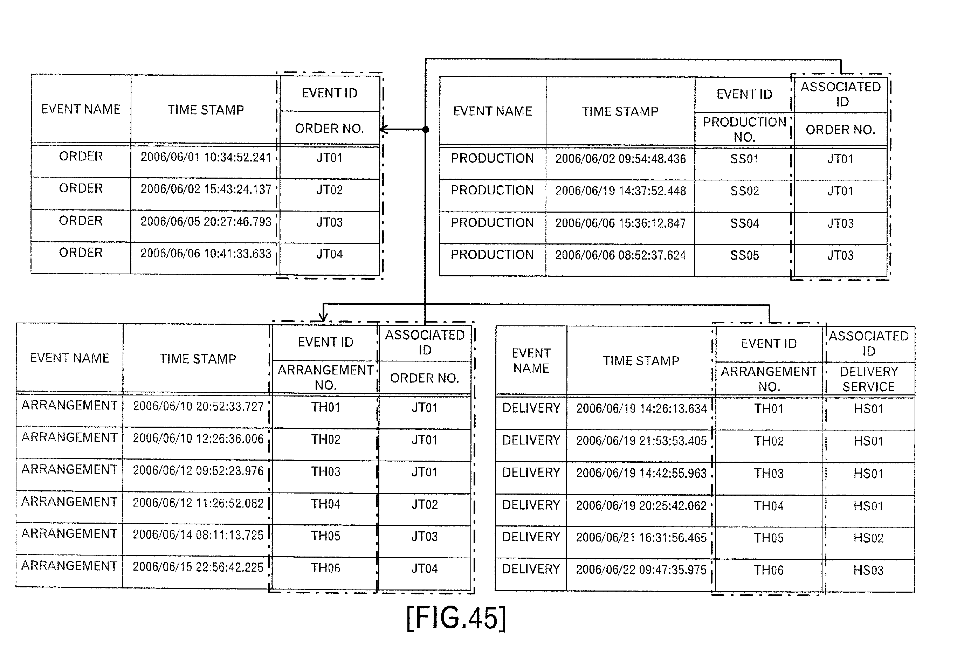

Fig. 45 is a diagram depicting an example of the event data and inter-event relational tree;

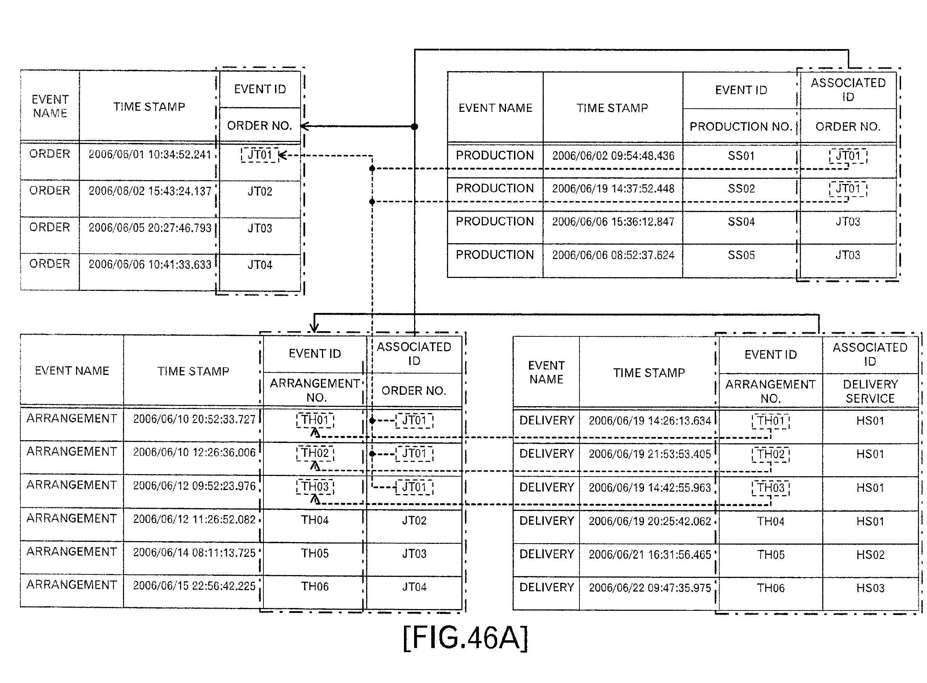

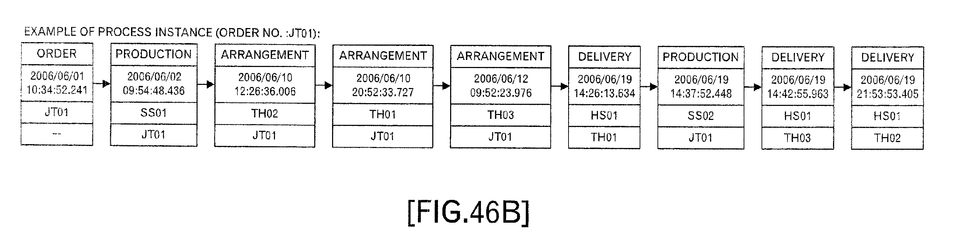

Fig. 46A and 46B are diagrams to explain process instance generation from the event data;

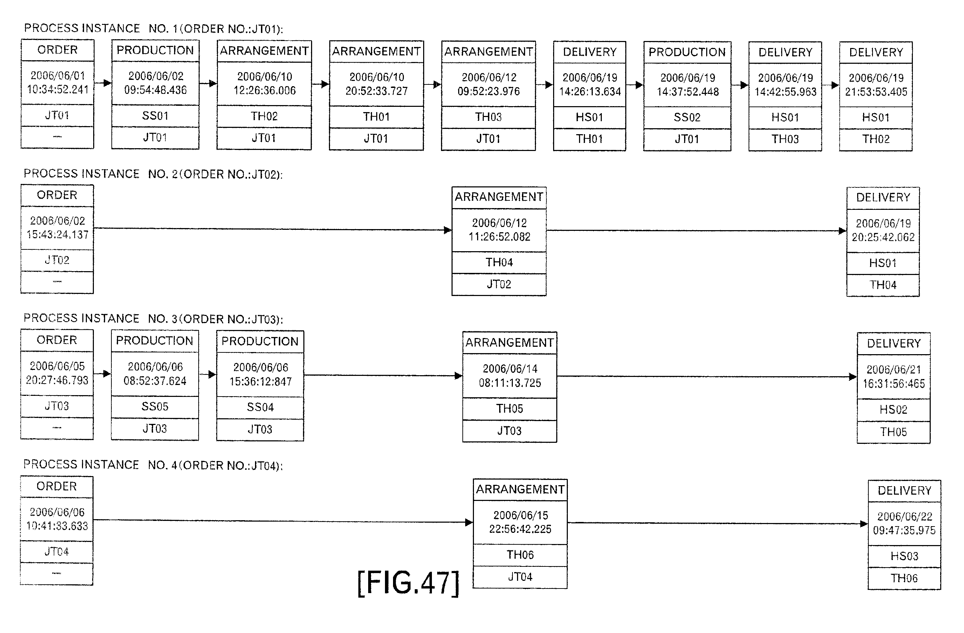

Fig. 47 is a diagram depicting an example of process instances;

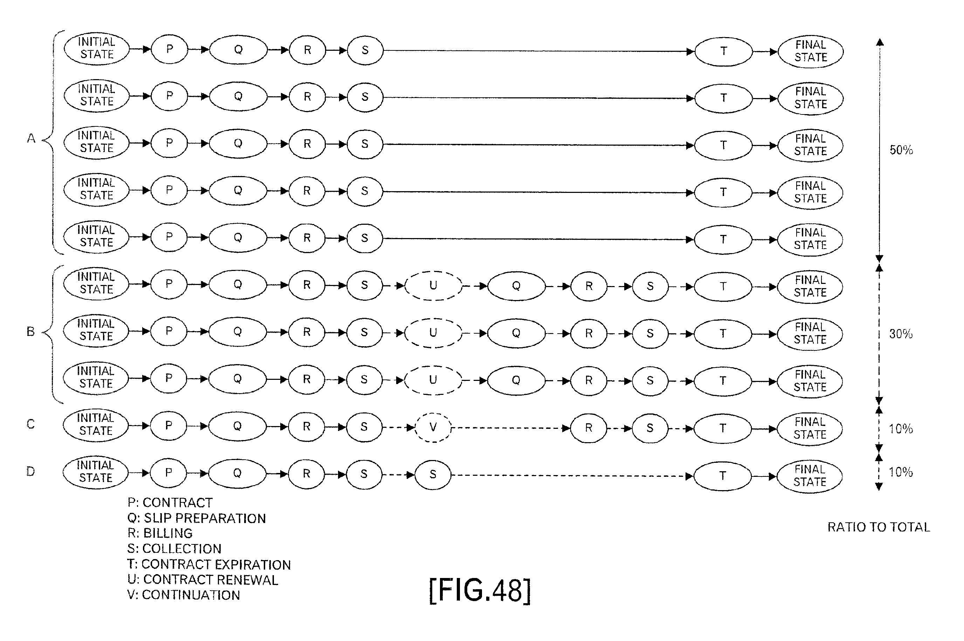

Fig. 48 is a diagram to explain an extraction processing of main and exceptional flows;

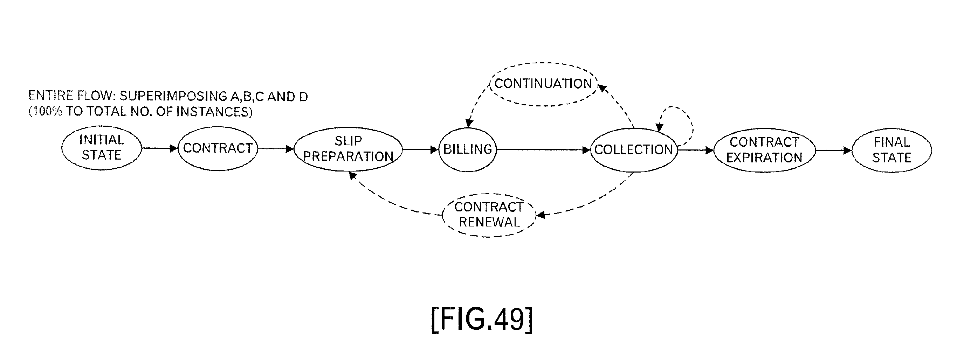

Fig. 49 is a diagram representing a display example in case where the process instances depicted in Fig. 48 are superimposed;

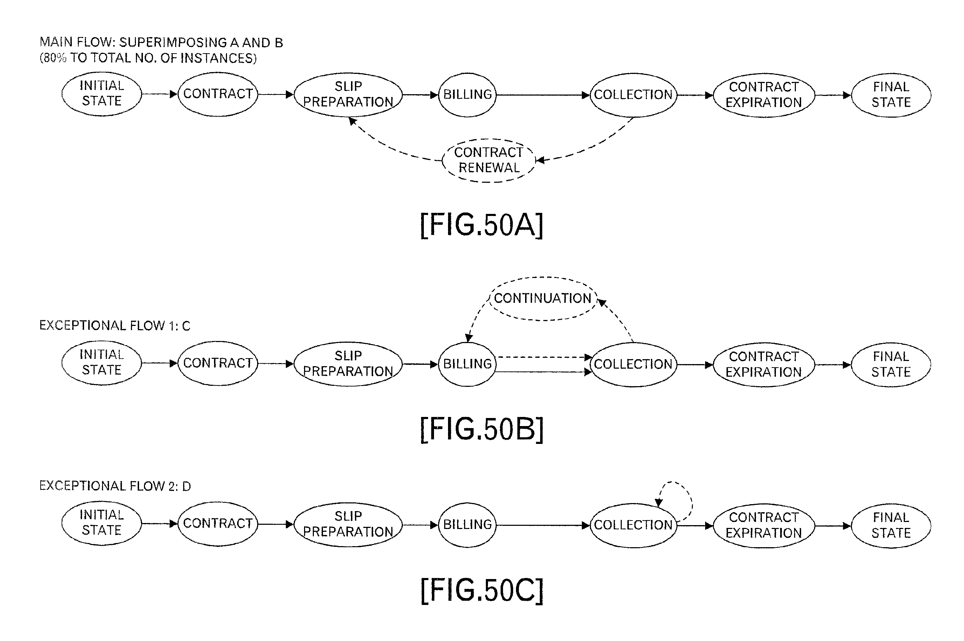

Figs. 50A to 50C are diagrams depicting a display example in case where the process instances depicted in Fig. 48 are categorized into the main flow and exceptional flows;

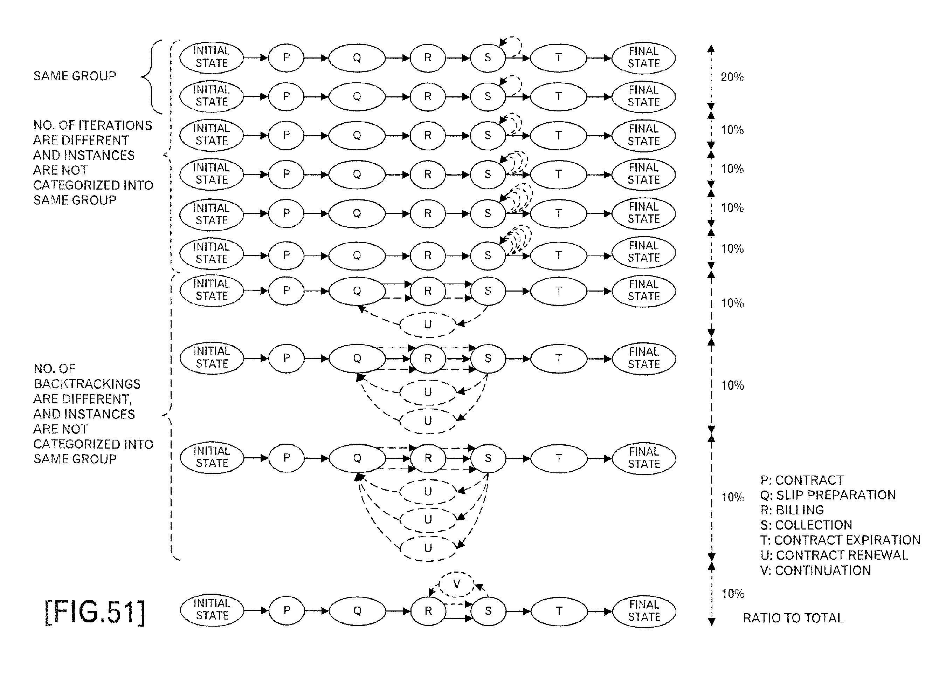

Fig. 51 is a diagram depicting an example of the process instances to explain a repetition cancellation processing;

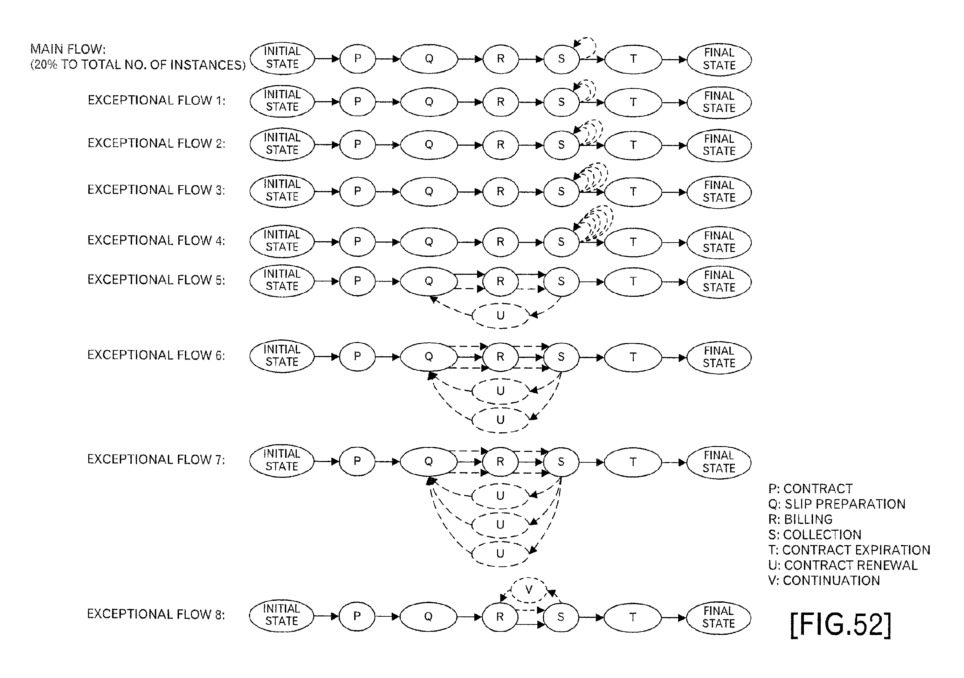

Fig. 52 is a diagram depicting an example in case where the process instances depicted in Fig. 51 are simply categorized;

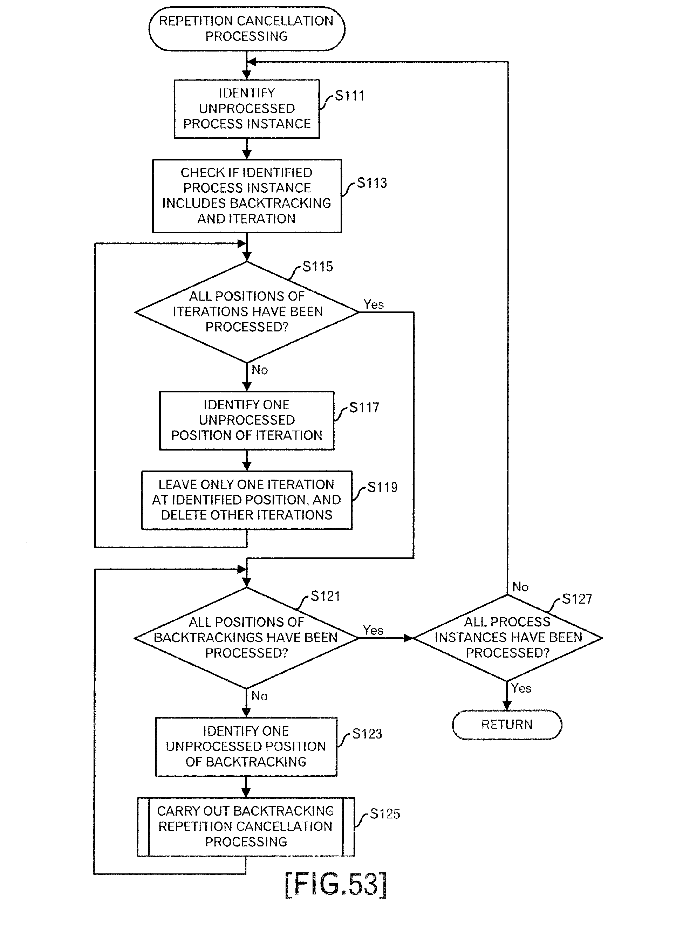

Fig. 53 is a diagram depicting a processing flow of the repetition cancellation processing;

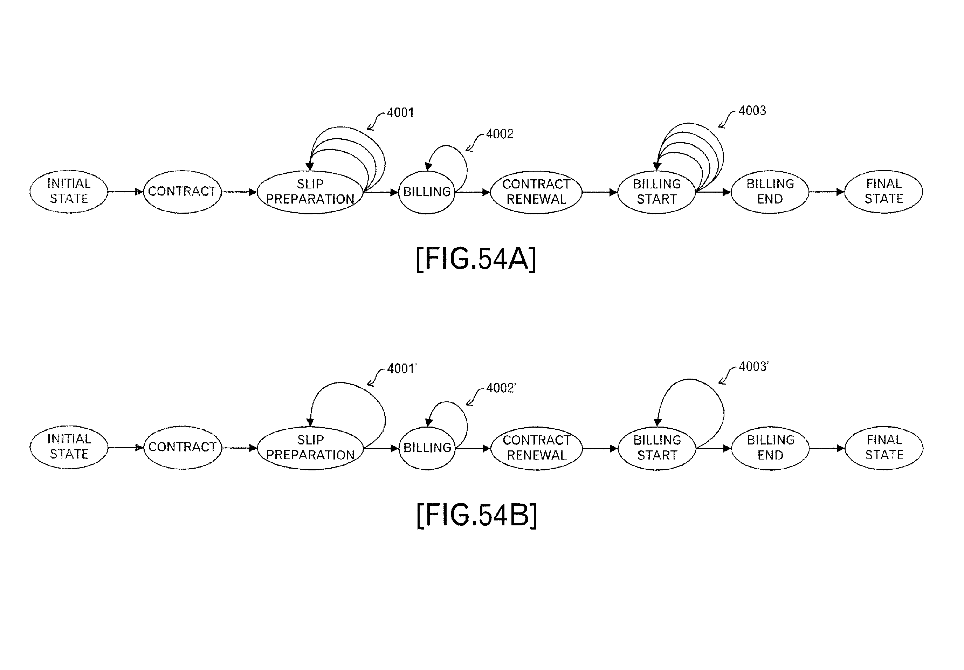

Fig. 54A is a diagram depicting an example of process instances having repeated iterations;
Fig. 54B is a diagram depicting an example of process instances in case where the additional iteration is deleted;

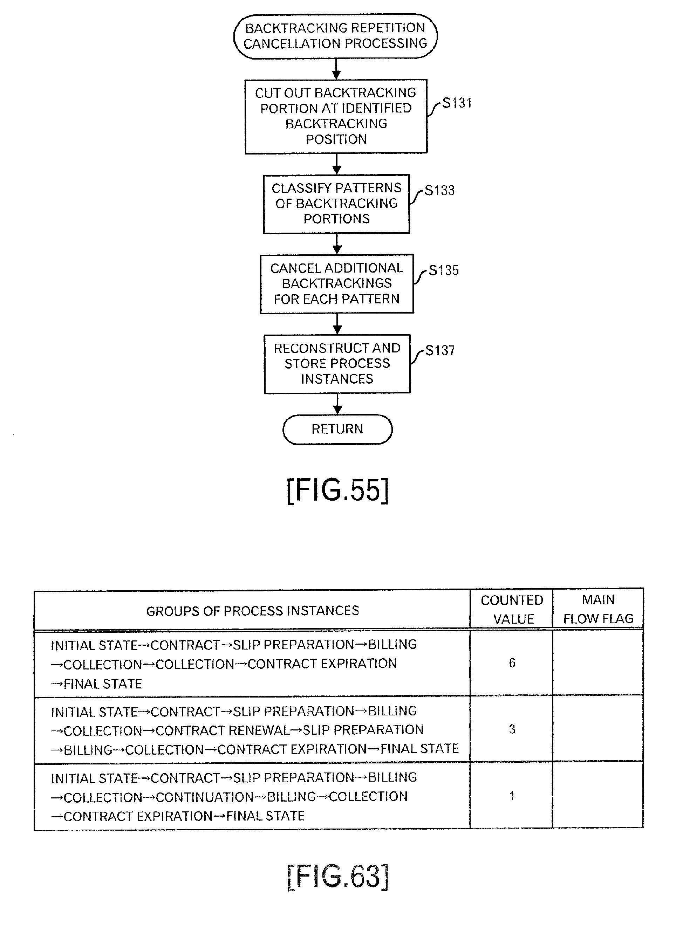

Fig. 55 is a diagram depicting a processing flow of a backtracking repetition cancellation processing;

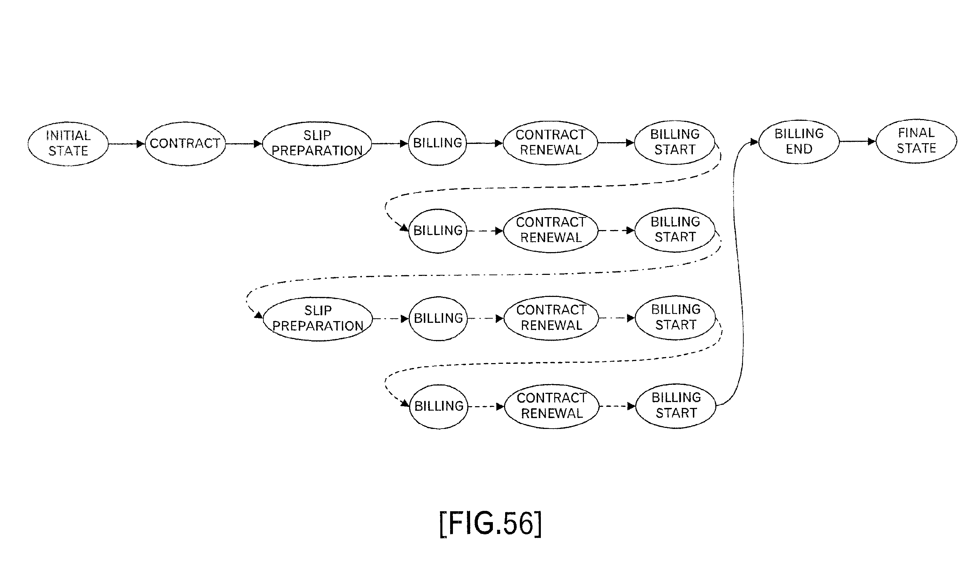

Fig. 56 is a diagram depicting an example of process instances to explain the backtracking repetition cancellation processing;

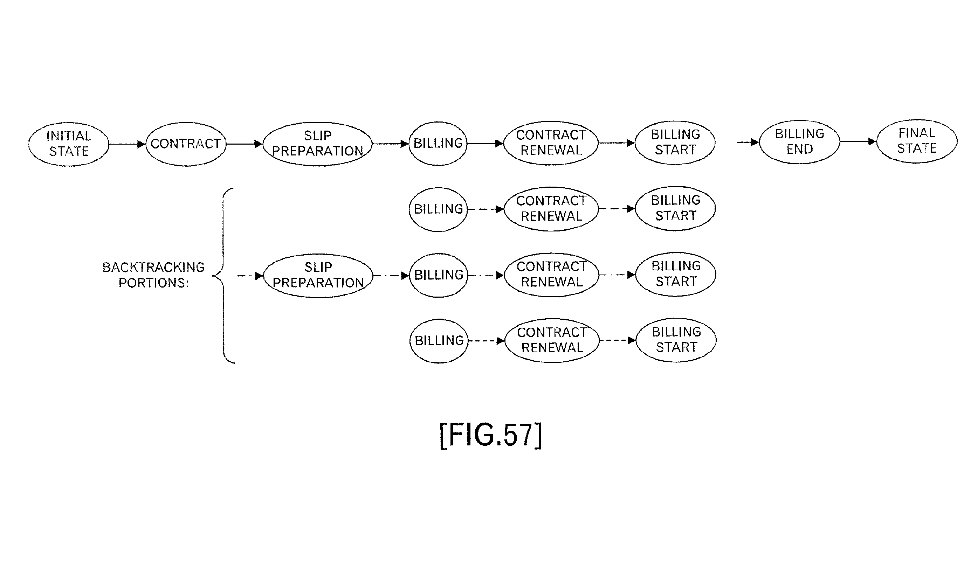

Fig. 57 is a diagram to explain cut-out of the backtracking portion;

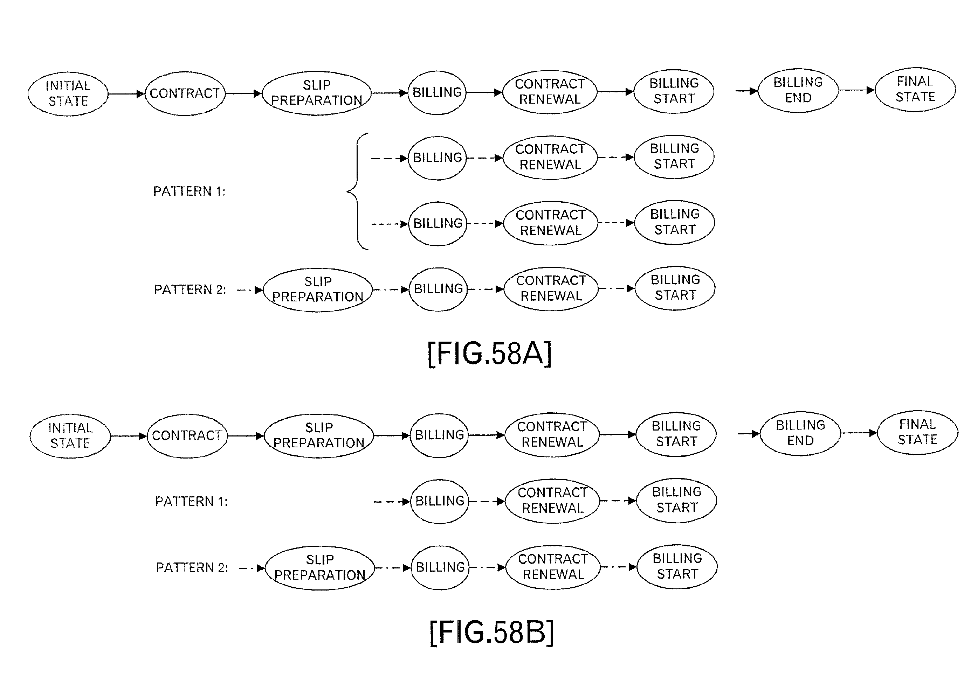

Fig. 58A is a diagram to explain classification of the backtracking portions;
Fig. 58B is a diagram to explain a processing to delete repetition of the backtracking portions;

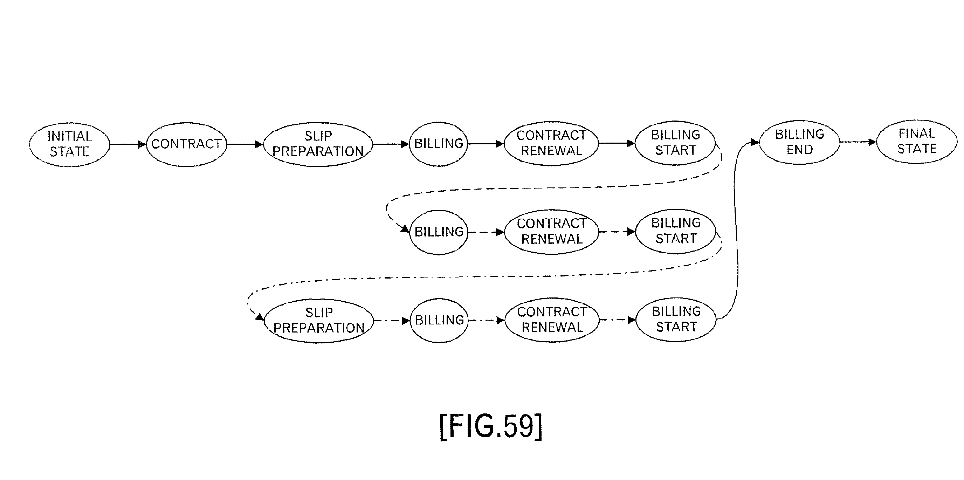

Fig. 59 is a diagram depicting a reconstruction example of the process instances;

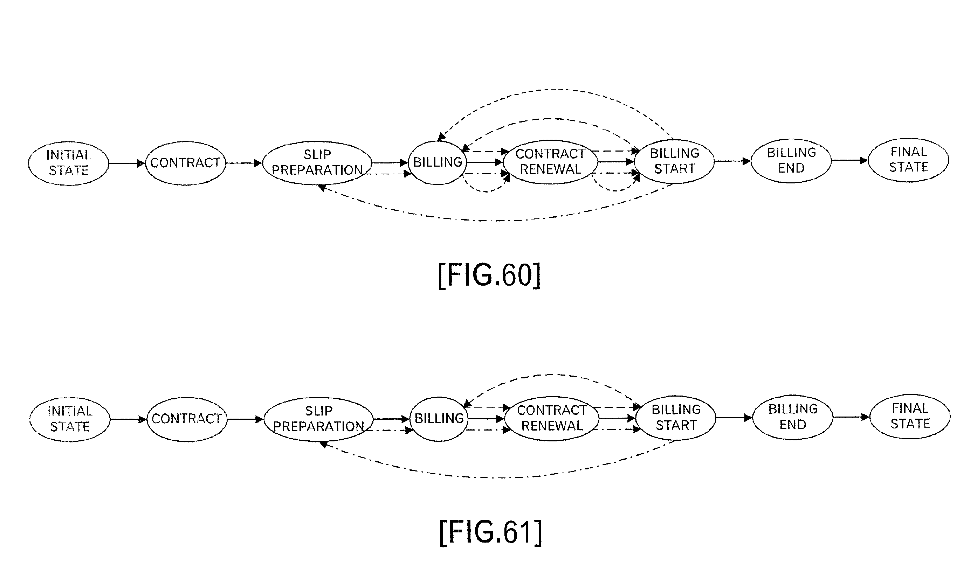

Fig. 60 is a diagram depicting an example of superimposing display of the process instances in Fig. 56;
Fig. 61 is a diagram depicting an example of superimposing display of the process instances in Fig. 59;

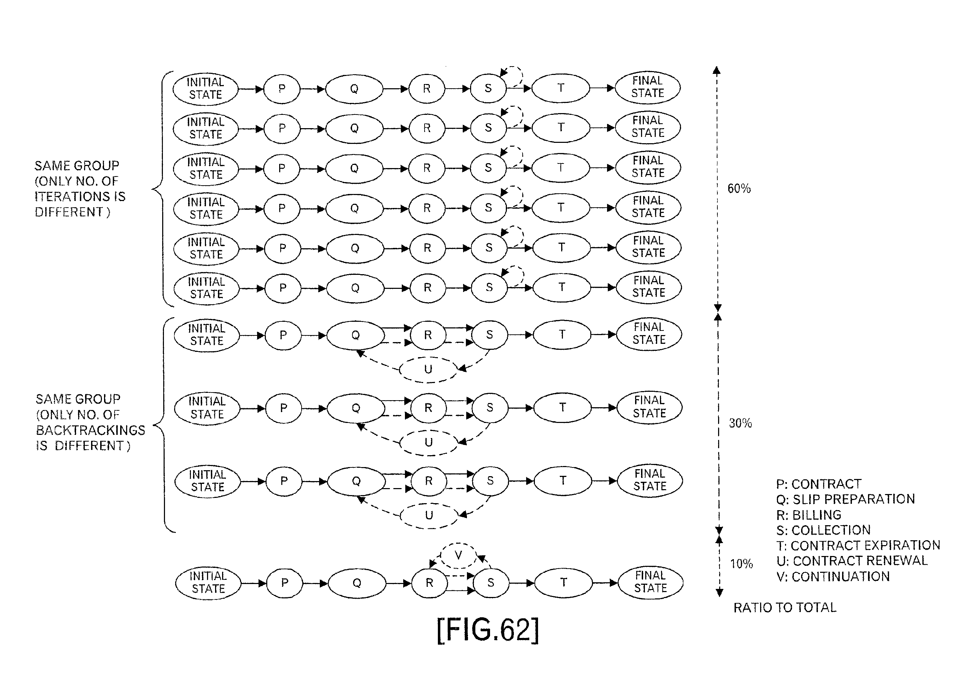

Fig. 62 is a diagram depicting process instances after carrying out the repetition cancellation processing for the example of the process instances depicted in Fig. 51;
Fig. 63 is a diagrams depicting an example of data stored in the model data storage;

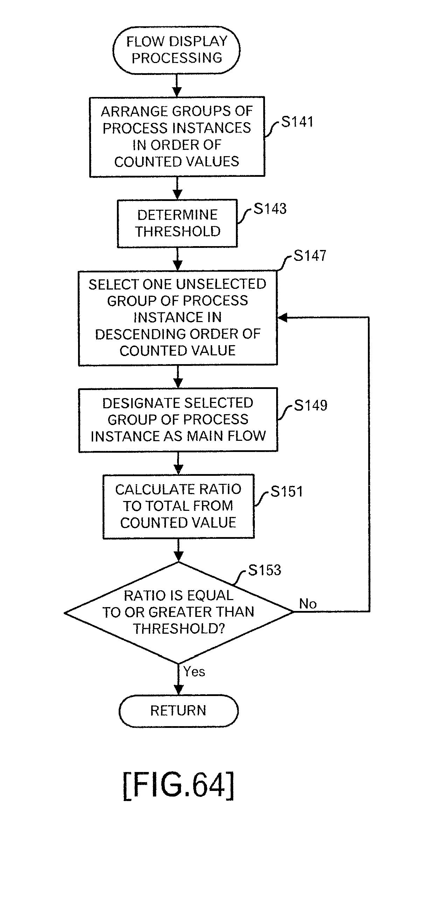

Fig. 64 is a diagram depicting a processing flow of a flow display processing;

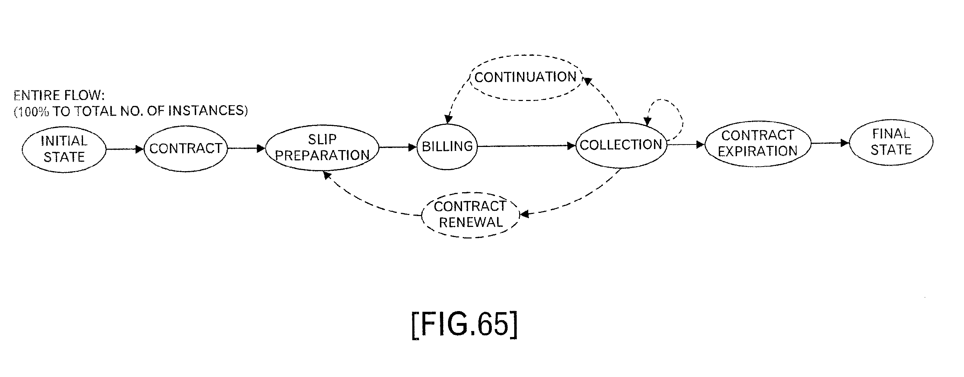

Fig. 65 is a diagram depicting a display example in case where all process instances registered in Fig. 63 are superimposed;

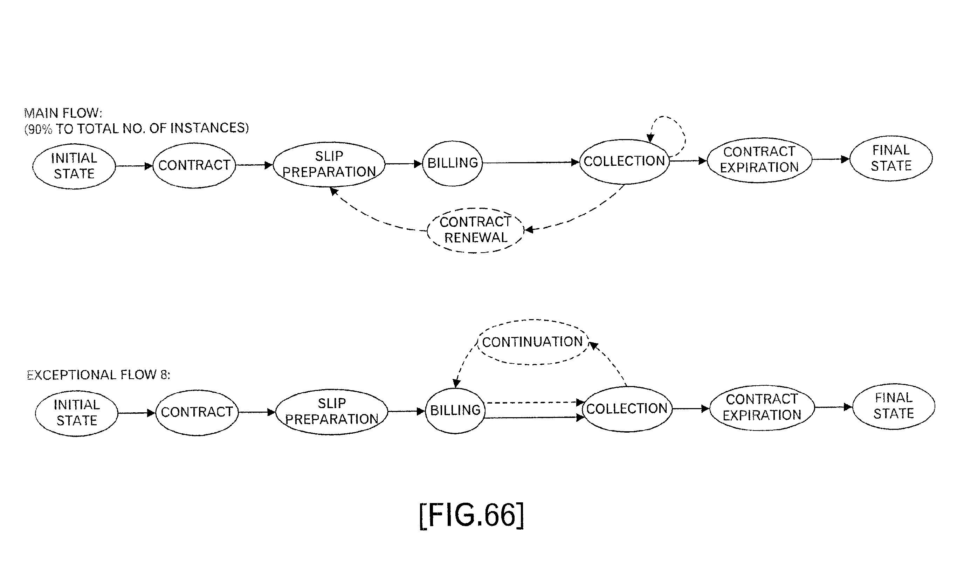

Fig. 66 is a diagram depicting a display example in case of separating the process instances registered in Fig. 63 into the main flow and the exceptional flow; and

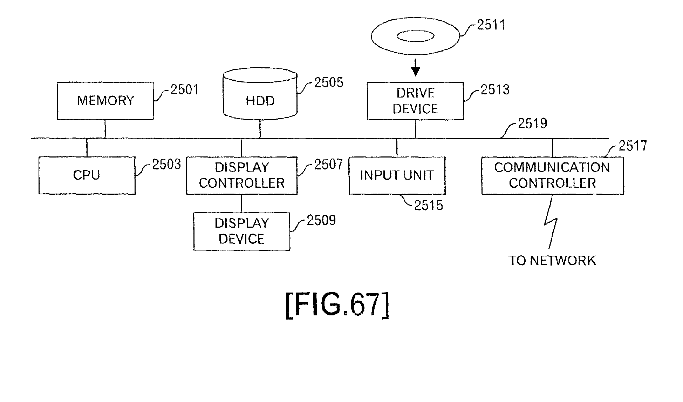

Fig. 67 is a functional block diagram of a computer.

### Best mode for carrying out the invention

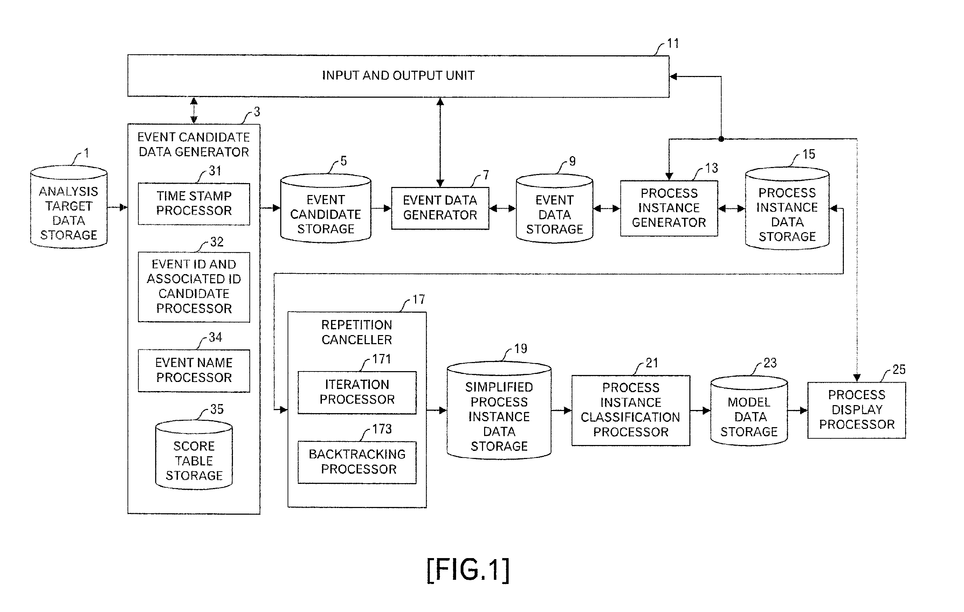

Fig. 1 depicts a functional block diagram of a business system analysis apparatus relating to one embodiment of this invention. The business system analysis apparatus relating to this embodiment includes an analysis target data storage 1 storing data (records of databases, log data, records of network DBs (NDBs), journals and the like, which are generated in a predetermined period) collected from one or plural analysis target systems; an event candidate data generator 3 that generates event candidate data from the analysis target data storage 1; an event candidate data storage 5 that stores the event candidate data generated by the event candidate data generator 3; an input and output unit 11, which is an interface with a user; an event data generator 7 that accepts user's instructions through the input and output unit 11 and generates event data; an event data storage 9 that stores the event data generated by the event data generator 7; a process instance generator 13 that generates process instances from the event data stored in the event data storage 9; a process instance data storage 15 that stores data of the process instances generated by the process instance generator 13; a repetition canceller 17 that carries out a processing to delete trackbackings and iterations, which make it difficult to grasp the entire image of the business, by using data of the process instances stored in the process instance data storage 15; a simplified process instances data storage 19 that stores data of the process instances processed by the repetition canceller 17; a process instance classification processor 21 that classifies the process instances stored in the simplified process instance data storage 19 into process types and counts, for each process type, the number of appearances of the process instances; a model data storage 23 that stores processing results of the process instance classification processor 21; and a process display processor 25 that carries out a processing required for display of the business flow by using data stored in the model data storage 23.

Incidentally, the input and output unit 11 operates, as the interface with the user, for the event candidate data generator 3, the process instance generator 13 and the process display processor 25. In addition, each processor may carry out a processing such as reading out processing results and the like to present the user with the read data through the input and output unit 11.

In addition, the event candidate data generator 3 has a time stamp processor 31, an event ID and associated ID candidate processor 32; an event name processor 34 and a score table storage 35. Furthermore, the repetition canceller 17 has an iteration processor 171 and a backtracking processor 173.

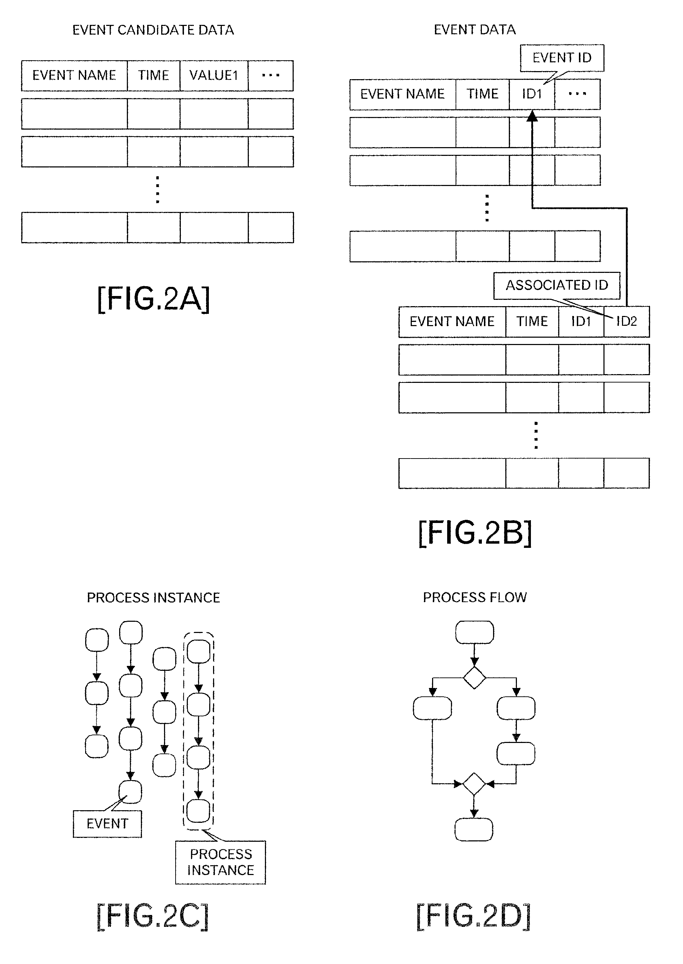

Next, rough processing contents of the business system analysis apparatus will be explained by using Figs. 2A to 2D. First, the event candidate data generator 3 generates event candidate data from data for the business systems, which is stored in the analysis target data storage 1. An example of the event candidate data is depicted in Fig. 2A. In the example in Fig. 2A, records each including an event name, a time (a time stamp, which is an occurrence time of the event), a first value (value 1) other than the time, a second value (value 2) other than time and the like are extracted from, for example, one table (e.g. a database). Namely, data fields that are candidates of the event name and the time stamp, and further an event ID and an associated ID are identified.

Next, the event data generator 7 generates event data from the event candidate data stored in the event candidate data storage 5. An example of the event data is depicted in Fig. 2B. In the example of Fig. 2B, records respectively including the event name, the time (the time stamp which is the occurrence time of the event), the event ID (here, ID1) and other values and records respectively including the event name, the time (the time stamp), ID1, ID2 and the like are extracted from plural table (e.g. databases), and when one value of the field values of the ID1, which is the event ID of the record of a first event class (i.e. event type) is used as one of the field values of the ID2, which is an associated ID of the record of a second event class (i.e. event type), it is identified that each of the records (i.e. event instances) of the second event class is associated with a specific record (i.e. event instance) of the first event class. Such a processing itself for extracting the association between the events is not a main portion of this embodiment, and for example, is disclosed in Japanese Patent Application 2006-197294 (filed on July 19, 2006) and its counterpart foreign applications, and those contents are incorporated into this application.

After that, the process instance generator 13 generates data of the process instances from the event data stored in the event data storage 9. An example of the process instance is depicted in Fig. 2C. In the example of Fig. 2C, four process instances are depicted as examples, and a series of event instances (specific events) are included in the respective process instances. Namely, a process instance includes a series of event instances (i.e. specific events respectively corresponding to specific records), which belong to the event class, such as "order", "slip issuance", "delivery" and "inspection". However, it is unnecessary that the event instances included in the process instance are originated from all of the event class, and plural event instances belonging to one event class may be included. Incidentally, the generation processing itself of the process instances is not a main portion in this embodiment, and for example, a business process tracking method described in U.S. Patent Application Publication 2005/076059A1 or the like can be adopted. Incidentally, this publication is incorporated into this application.

Then, data of the process instances is processed by the repetition canceller 17 and the process instance classification processor 21, and the process display processor 25 generates data of the process flow (also called business flow) from data stored in the model data storage 23 to display the generated data to a display device through the input and output unit 11. An example of the process flow is depicted in Fig. 2D. In the example of Fig. 2D, a business flow generated by aggregating the process instances is depicted.

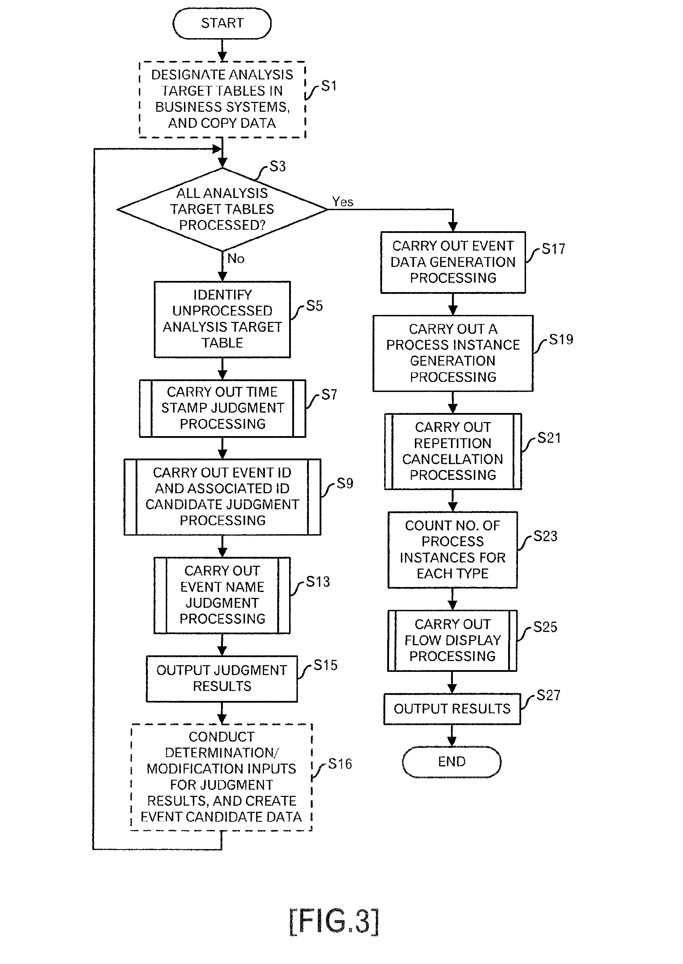

Next, the detailed processing of the business system analysis apparatus depicted in Fig. 1 will be explained by using Figs. 3 to 66. First, the user designates analysis target tables in the business systems, and makes their data copied and stored into the analysis target data storage 1 (Fig. 3: step S1). For example, an order DB, a production DB, an arrangement DB, a delivery DB and a product number DB are designated, and records generated and stored in a predetermined period are copied and stored into the analysis target data storage 1. Incidentally, when these DBs are relational databases, the schema information is copied and stored into the analysis target data storage 1. Because this step is a processing conducted in advance by the user instructing a computer, this step is depicted by a dotted line block in Fig. 3.

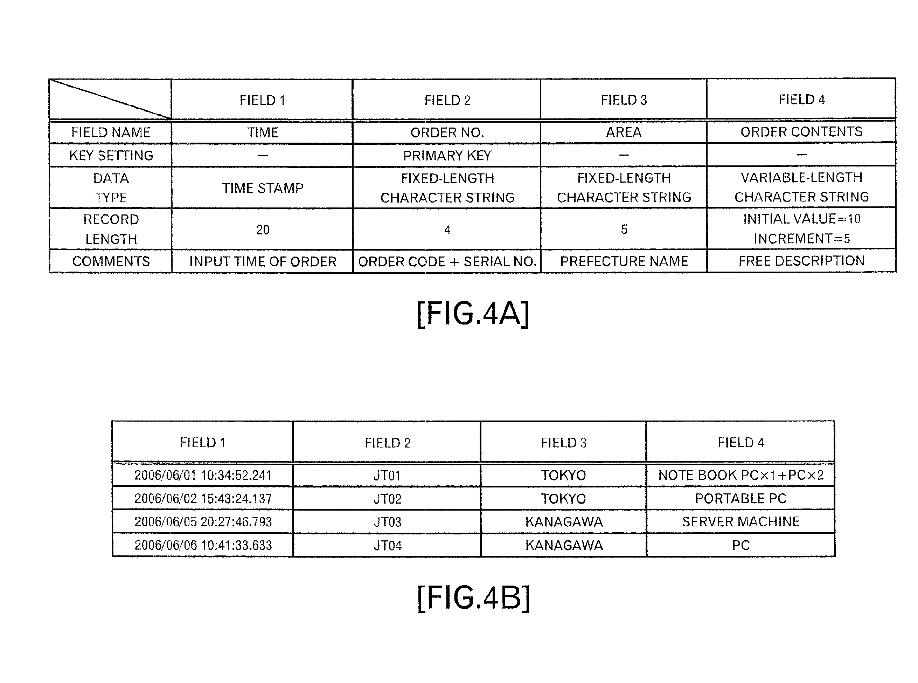

For example, when the order DB is the relational database, the schema information as depicted in Fig. 4A and records as depicted in Fig. 4B are stored into the analysis target data storage 1. In the example of the schema information depicted in Fig. 4A, for each of the fields 1 to 4, the field name, key setting data, data type, record length and comments are registered. It is understood from Fig. 4A that the time is registered in the field 1, the order number, which is a main key, is registered into the field 2, the area is registered in the field 3 and the order contents are registered in the field 4. Specifically, the records as depicted in Fig. 4B is stored, and when the schema information as depicted in Fig. 4A is obtained, contents of the records as depicted in Fig. 4B can easily be interpreted.

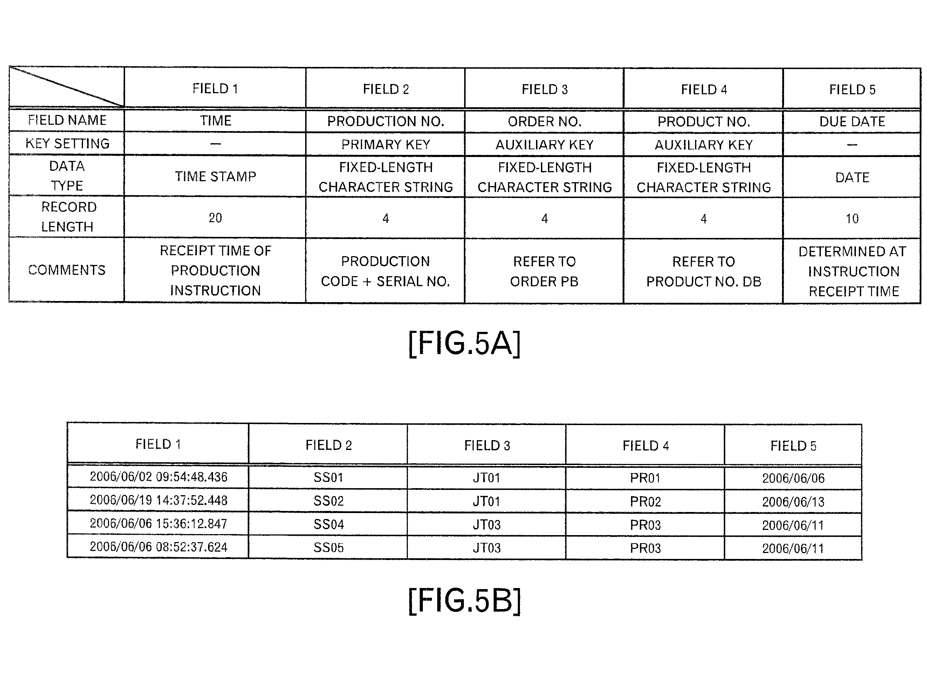

Similarly, when the production DB is the relational database, the schema information as depicted Fig. 5A and the records as depicted in Fig. 5B are stored into the analysis target data storage 1. In the example of the schema depicted in Fig. 5A, for each of the fields 1 to 5, the fields name, key setting data, data type, record length and comments are registered. It is understood from Fig. 5A that the time is registered in the field 1, the production number, which is a main key, is registered in the field 2, an order number, which is an auxiliary key, is registered in the field 3, the product number, which is a auxiliary key, is registered in the field 4 and the due date is registered in the field 5. Specifically, the records as depicted in Fig. 5B are stored, and when the schema information as depicted in Fig. 5A is obtained, the contents of the records as depicted in Fig. 5B can easily be interpreted.

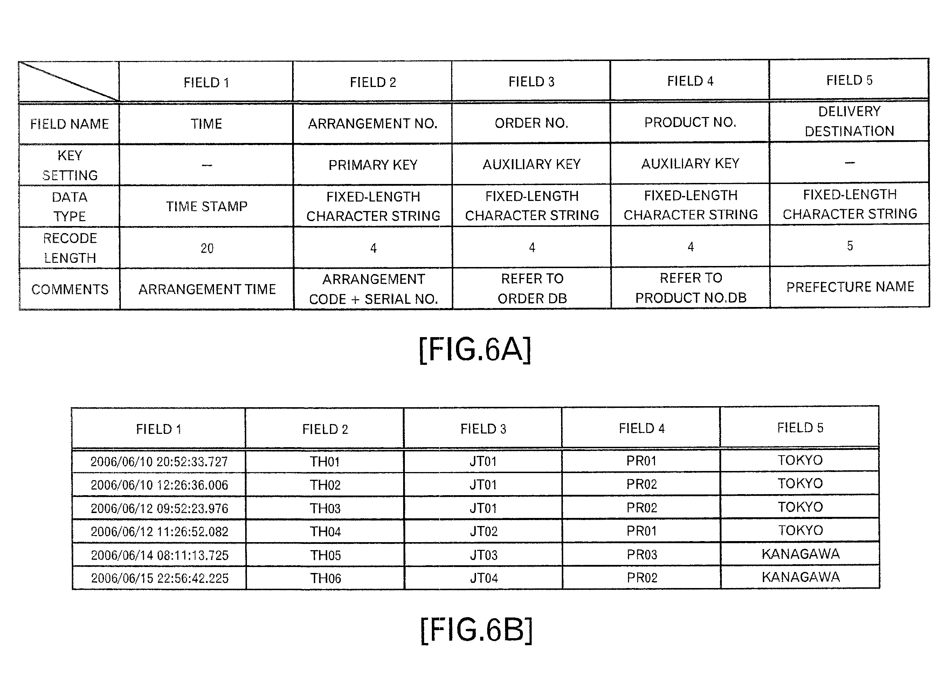

In addition, when the arrangement DB is the relational database, the schema information as depicted in Fig. 6A and records as depicted in Fig. 6B are stored into the analysis target data storage 1. In the example of the schema information depicted in Fig. 6A, for each of the fields 1 to 5, the field name, key setting data, data type, record length and comments are registered. It is understood from Fig. 6A that the time is registered in the field 1, the arrangement number, which is a main key, is registered in the field 2, the order number, which is an auxiliary key, is registered in the field 3, the production number, which is the auxiliary key, is registered in the field 4 and a delivery destination is registered in the field 5. Specifically, the records as depicted in Fig. 6B are stored, and when the schema information as depicted in Fig. 6A are obtained, the contents of the records as depicted in Fig. 6B can easily be interpreted.

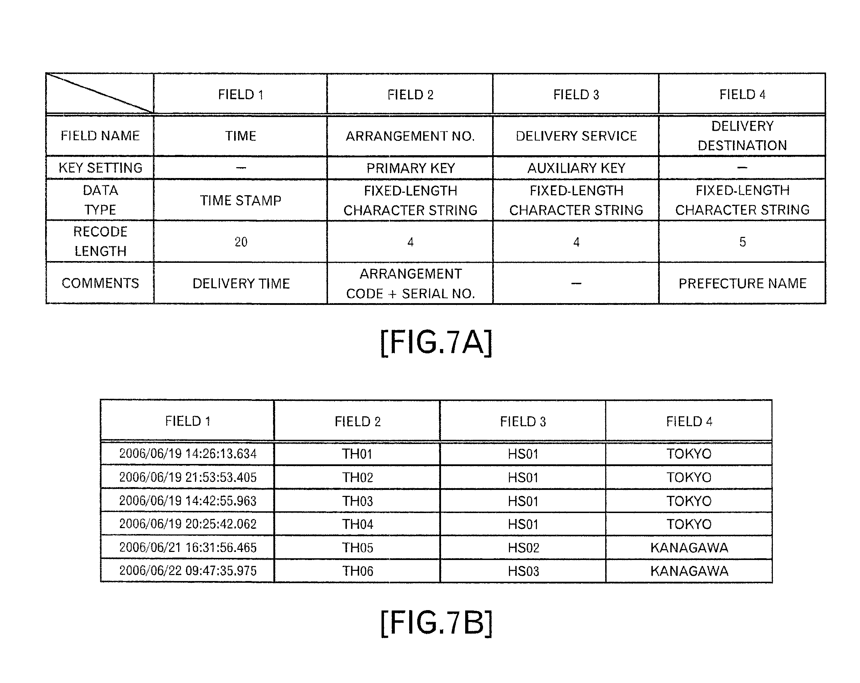

Furthermore, when the delivery DB is the relational database, the schema information as depicted in Fig. 7A and records as depicted in Fig. 7B are stored in the analysis target data storage 1. In the example of the schema information as depicted in Fig. 7A, for each of the fields 1 to 4, the fields name, key setting data, data type, record length and comments are registered. It is understood from Fig. 7A that the time is registered in the field 1, the arrangement number, which is a main key, is registered in the field 2, the delivery service, which is the auxiliary key, is registered in the field, 3, and the delivery destination is registered in the field 4. Specifically, the records as depicted in Fig. 7B are stored, and when the schema information as depicted in Fig. 7A is obtained, the contents of the records as depicted in Fig. 7B can easily be interpreted.

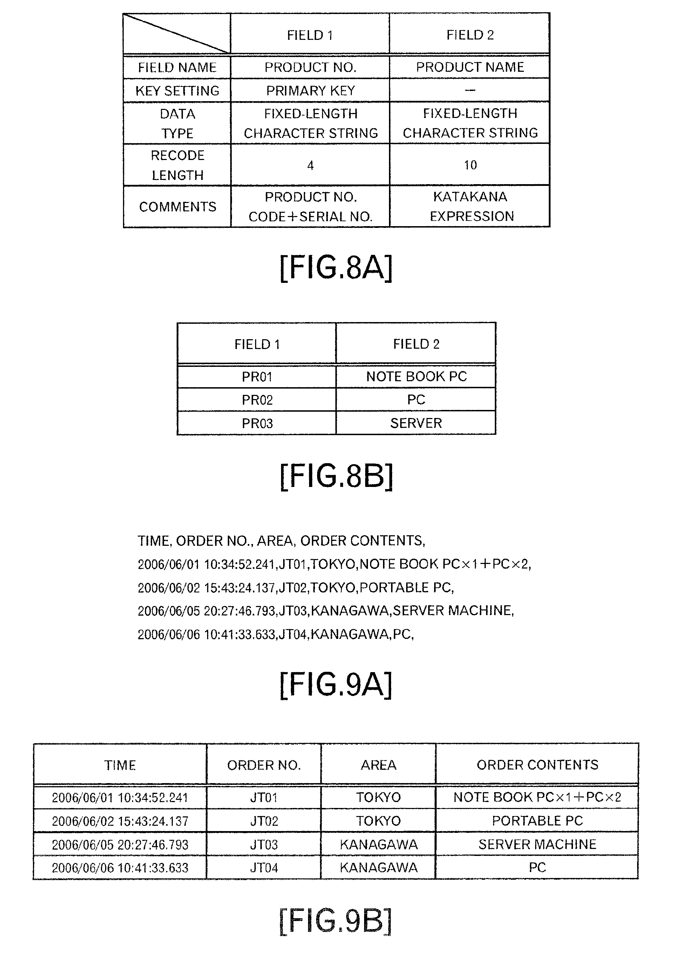

Moreover, when the product number DB is the relational database, the schema information as depicted in Fig. 8A and records as depicted in Fig. 8B are stored in the analysis target data storage 1. In the example of the scheme information depicted in Fig. 8A, for each of the fields 1 and 2, the field name, key setting data, data type, record length and comments are registered. It is understood from Fig. 8A that the product number, which is the main key, is registered in the field 1, and the product name is registered in the field 2. Specifically, the records as depicted in Fig. 8B are stored, and when the schema information as depicted in Fig. 8A is obtained, the contents of the records as depicted in Fig. 8B can easily be interpreted.

On the other hand, when data of the order DB is obtained in the CSV format, data as depicted in Fig. 9A is stored into the analysis target data storage 1. In the example of Fig. 9A, label data such as the time, the order number, the area and the order contents is included in the header, and after the header, data is enumerated in an order of the label, and data is sectioned by commas. When the data format of Fig. 9A is converted to a table format in order to make Fig. 9A easily understood, Fig. 9B is obtained. Namely, a table including a column of the time, a column of the order number, a column of the area and a column of the order contents is obtained. Because there is no schema information, all data is stored as character strings. In addition, there is no key setting data.

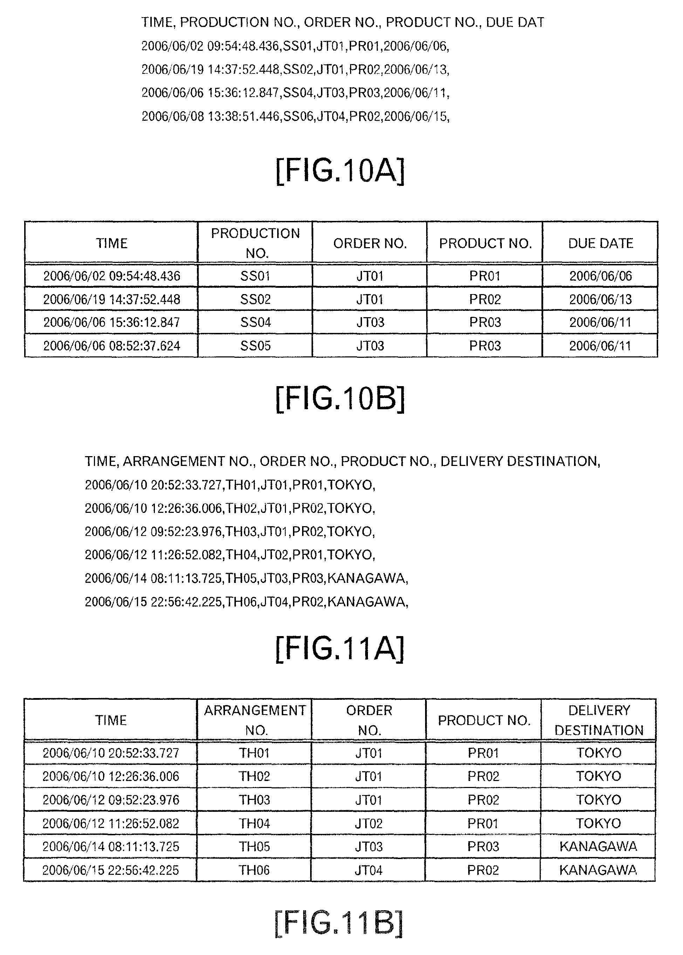

Similarly, when data of the production DB is obtained, data as depicted in Fig. 10A is stored in the analysis target data storage 1. In the example of Fig. 10A, the label data of the time, the production number, the order number, the product number and the due date is contained in the header, and after the header, data is enumerated in an order of the label, and data is sectioned by commas. When a data format is converted into a table format in order to make Fig. 10A easily understood, a table as depicted in Fig. 10B is obtained. Namely, the table includes a column of the time, a column of the production number, a column of the order number, a column of the product number and a column of the due date.

In addition, when data of the arrangement DB is obtained in the CSV format, data as depicted in Fig. 11A is stored into the analysis target data storage 1. In the example of Fig. 11A, the label data of the time, the arrangement number, the order number, the product number and the delivery destination is contained in the header, and after the header, data is enumerated in an order of the label, and data is sectioned by commas. When the data format is converted into the table format in order to make Fig. 11A easily understood, a table as depicted in Fig. 11B is obtained. Namely, the table including a column of the time, a column of the arrangement number, a column of the order number, and a column of the product number and a column of the delivery destination is obtained.

Furthermore, when data of the delivery DB is obtained in the CSV format, data as depicted in Fig. 12A is stored into the analysis target data storage 1. In the example of Fig. 12A, the label data of the time, the arrangement number, the delivery service and the delivery destination is contained in the header, and after the header, data is numerated in an order of the label, and data is sectioned by commas. When the data format is converted into the table format in order to make Fig. 12A easily understood, a table as depicted in Fig. 12B is obtained. Namely, the table including a column of the time, a column of the arrangement number, a column of the delivery service and a column of the delivery destination is obtained.

In addition, when data of the product DB is obtained in the CSV format, data as depicted in Fig. 13A is stored into the analysis target data storage 1. In the example of Fig. 13A, the label data of the product number and the product name is contained in the header, and after the header, data is enumerated in an order of the label, and data is sectioned by commas. When the data format is converted into the table format in order to make Fig. 13A easily understood, a table as depicted in Fig. 13B is obtained. Namely, the table including a column of the product number and a column of the product name is obtained.

For example, the event candidate data generator 3 of the business system analysis apparatus judges whether or not all of the analysis target tables have been processed (step S3) . When an unprocessed analysis target table exists, the event candidate data generator 3 identifies one unprocessed analysis target table (step S5). Then, the event candidate data generator 3 carries out a time stamp judgment processing (step S7). This time stamp judgment processing will be explained by using Figs. 14 and 15.

First, the time stamp processor 31 of the event candidate data generator 3 identifies one unprocessed field in the analysis target table by referring to the analysis target data storage 1 (Fig. 14: step S31). Then, the time stamp processor 31 judges whether or not the schema information of the analysis target table can be used in the analysis target data storage 1 (step S33)_{.}

When the schema information can be used, the time stamp processor 31 identifies a data portion for a processing target field in the schema information, and judges whether or not a data type of the processing target field in the identified data portion is a time stamp type (step S35). When the data type of the processing target field is not the time stamp type, the processing shifts to step S39. For example, when data as depicted in Figs. 9A to 13A is processed, there is no scheme information. Therefore, the processing shifts to the step S39.

On the other hand, when it is judged that the data type of the processing target field is the time stamp type, the time stamp processor 31 sets "determined" to time stamp judgment of the processing target field, and stores the time stamp judgment data into a storage device such as a main memory (step S37). Then, the processing shifts to step S43.

For example, in case of the schema information as depicted in Fig. 4A, because the data type of the field 1 is the time stamp type, the time stamp judgment "determined" is set when the field 1 is the processing target field. In case of the schema information as depicted in Fig. 5A, because the data type of the field 1 is the time stamp type, the time stamp judgment "determined" is set when the field 1 is the processing target field. The same matters can also be applied to Figs. 6A and 7A. In case of Fig. 8A, for all fields, the processing shifts from the step S35 to the step S39.

When it is judged at the step S33 that the schema information cannot be used, or when the data type of the processing target filed is not the time stamp type, the time stamp processor 31 identifies a confidence degree based on a pertinent data portion of the processing target field in the schema information, label data representing the field name of the processing target field and the field value of the processing target field by referring to a time stamp confidence score table stored in the score table storage 35 (step S39).

An example of the time stamp confidence score table is depicted in Fig. 15. In the example of Fig. 15, 1% is set as the confidence score when the data type of the field is the variable length character string, 5% is set as the confidence score when the data type of the field is real, 90% is set as the confidence score when the end of the field name is "time" or the like, 70% is set as the confidence score when the end of the field name is "date", "day" or the like and does not contain "time" or the like, 10% is set as the confidence score when a word or phases representing a future timing such as "plan", "due date" or the like is designated, 5% is set as the confidence score when the character string of the field value contains a character other than characters associated with the time, such as the name of an era (e.g. symbol), "/", ":", "'", ".", "-", numeral, space and the like, 90% is set as the confidence score when the character string in the field value is in a format "YYYY/MM/DD hh:mm:ss", 70% is set as the confidence score when the character string of the field value is in a format "YYYY/MM/DD", 30% is set as the confidence score when the same field values are contained in the field, and 50% is set as the confidence score when there is no pertinent item in the score table.

For example, in case of the schema information as depicted in Fig. 4A and the records as depicted in Fig. 4B, the confidence score 5% is identified for the field 2, because the character other than the characters associated with the time is contained in the field values. Similarly, the confidence score 5% is also identified for the field 3, because the character other than the characters associated with the time is contained in the field values. Furthermore, the confidence score 1% is identified for the field 4, because the data type is the variable length character string. Incidentally, because the character other than the characters associated with the time is contained in the field values of the field 4, plural items in the time stamp confidence score table are applicable to the field 4. In such a case, a value, which is further away from the central value 50%, is adopted in this embodiment. Namely, the confidence score 1% is adopted rather than the confidence score 5% applicable when the field value contains the character other than the characters associated with the time.

On the other hand, in case of Fig. 9A, in which the schema information does not exist, the confidence score 90% is identified for the field 1, because the character string of the field value is in the format "YYYY/MM/DD hh:mm: ss". The fields 2 and 3 are the same as the case of Figs. 4A and 4B. However, the confidence score 5% is identified for the field 4, because the data type of the field cannot be identified and it is judged that the field value contains the character other than the characters associated with the time.

Moreover, in case of the schema information as depicted in Fig. 5A and the records as depicted in Fig. 5B, the confidence score 5% is identified for the fields 2 to 4, because the field value contains the character other than the characters associated with the time. Because the character string of the field name in the field 5 contains "due date", the confidence score 10% is identified for the field 5. Incidentally, because the character string of the field value in the field 5 is in the format "YYYY/MM/DD", plural items in the time stamp confidence score table are applicable to the field 5. In such a case, a value, which is further away from the central value 50%, is adopted in this embodiment. Namely, 10% is adopted rather than the confidence score 70% applicable when the character string of the field value is in the format "YYYY/MM/DD". In case of Fig. 10A, in which the schema information does not exist, the confidence score 90% is identified for the field 1, because the character string of the field value is in the format "YYYY/MM/DD hh:mm:ss". As for the fields 2 and 5, because the data type can not be identified, the same results as the case the schema information, exists are obtained.

Furthermore, in case of the schema information, as depicted in Fig. 6A and the records as depicted in Fig. 6B, the confidence score 5% is identified for the fields 2 to 5, because the field values include the character other than the characters associated with the time. In case of Fig. 11A, in which the schema information does not exist, the confidence score 90% is identified for the field 1, because the character string of the field value is in the format "YYYY/MM/DD hh:mm:ss". Because the data type can not be identified for the fields 2 and 5, the same results as the case where the schema information exists are obtained.

Moreover, in case of the schema information as depicted in Fig. 7A and the records as depicted in Fig. 7B, the confidence score 5% is identified for the fields 2 to 4, because the field values include the character other than characters associated with the time. In case of Fig. 12A in which the schema information does not exist, the confidence score 90% is identified for the field 1, because the character string of the field value is in the format "YYYY/MM/DD hh:mm:ss". Because the data type can not be identified for the fields 2 and 4, the same results as the case where the schema information exists are obtained.

Furthermore, in case of the schema information as depicted in Fig. 8A and the records as depicted in Fig. 8B, the confidence score 5% is identified for the fields 1 and 2, because the field values include the character other than the characters associated with the time. In case of Fig. 13A in which the schema information does not exist, because the data type can not be identified, the same results as the case where the schema information exists are obtained.

Returning to the explanation of Fig. 14, the time stamp processor 31 sets the identified confidence score to the time stamp judgment of the processing target field (step S41). The aforementioned numerical value is identified.

Then, the time stamp processor 31 judges whether or not all fields have been processed in the processing target table (step S43). When an unprocessed field exists, the processing returns to the step S31. On the other hand, when all of the fields have been processed, the processing returns to the calling source processing.

Thus, the greater confidence score is set to the field whose probability that the field corresponds to the time stamp of the event is high. Thus, the greater confidence score is set to the field whose probability that the field corresponds to the time stamp of the event is high. In addition, when it is apparent from the data type that the field corresponds to the time stamp, "determined" is set as data representing probability.

Returning to the explanation of Fig. 3, next, the event ID and associated ID candidate processor 32 carries out an event ID and associated ID candidate judgment processing (step S9). This event ID and associated ID candidate judgment processing will be explained in Figs. 16 and 17.

The event ID and associated ID candidate processor 32 identifies one unprocessed field in the analysis target table stored in the analysis target data storage 1 (step S51). Then, the event ID and associated ID candidate processor 32 judges whether or not field values of the processing target field are unique among all records (step S53). When the field values of the processing target field are not unique among all records, namely, records whose values in the processing target field are identical exist, the processing shifts to step S62.

Because the event ID is a storage field of the event identifier, the field values are never identical. Therefore, when the same values exist in the field of the event ID, it can be judged that the field value is not the event ID.

On the other hand, when the field values in the processing target field are unique among all records, the event ID and associated ID candidate processor 32 judges whether or not the field values of the processing target field, which are stored in the analysis target data storage 1, include NULL (step S55) . When the field values of the processing target field include "NULL", the processing shifts to the step S62. Because the event ID is the storage field of the event identifier, "NULL" never appears as the field value. When the field values of the processing target field are not unique among all records or when the field values of the processing target field include "NULL", the event ID and associated ID candidate processor 32 judges whether or not the number of kinds of the field values (from which "NULL" is excluded) of the processing target field is equal to or greater than 2 (step S62). When the number of kinds of the field values (from which "NULL" is excluded) of the processing target field is less than 2, the event ID and associated ID candidate processor 32 sets "denial" to event ID and associated ID candidate judgment, and stores the event ID and associated ID candidate judgment data into the storage device such as the main memory (step S63). Then, the processing shifts to the step S61. The associated ID is a value representing that a certain event corresponds to which other event. Therefore, when the number of kinds of the field values (from which "NULL" is excluded) is less than 2, any meaningful result cannot be obtained.

For example, in case of the table as depicted in Figs. 4B and 9B, as for each of the fields 1, 2 and 4, the same field, values do not exist, and as for the field 3, the same field, values exist. However, two or more kinds of field values other than "NULL" exist. Therefore, "denial" is not set to the event ID and associated ID candidate judgment for the fields 1 to 4.

In addition, in case of the table as depicted in Figs. 5B and 10B, as for the fields 1 and 2, the same field values do not exist, and as for the fields 3 to 5, the same field values exist. However, two or more kinds of field values other than "NULL" exist. Therefore, "denial" is not set to the event ID and associated ID candidate judgment for the fields 1 to 5.

Furthermore, in case of the table as depicted in Figs. 6B and 11B, as for the fields 1 and 2, the same field values do not exist, and as for the fields 3 to 5, the same field values exists. However two or more kinds of field values other than "NULL" exist. Therefore, "denial" is set to the event ID and associated ID candidate judgment for the fields 1 to 5.

Moreover, in case of the table as depicted in Figs 7B and 12B, as for the fields 1 and 2, the same field values do not exist, and as for the fields 3 and 4, the same field values exist. However two or more kinds of field values exist. Therefore, "denial" is set to the event ID and associated ID candidate judgment for the fields 1 to 4.

Furthermore, in case of the table as depicted in Figs. 8B and 13B, as for the fields 1 and 2, the same field values do not exist. Therefore, "denial" is not set to the event ID and associated ID candidate judgment for the fields 1 and 2.

When it is judged at the step S55 that the field values of the processing target field do not include "NULL", or when it is judged at the step S62 that the number of kinds of the field values of the processing target field is two or more, the event ID and associated ID candidate processor 32 identifies the confidence degree based on a pertinent data portion of the processing target field in the schema information, the label data representing the field name of the processing target field and the field values of the processing target field by referring to the event ID and associated ID candidate confidence score table stored in the score table storage 35 (step S57). However, when the pertinent item does not exist in the event ID and associated ID candidate confidence score table, the confidence score 50% is identified.

An example of the event ID and associated ID candidate confidence score table is depicted in Fig. 17. In the example of Fig. 17, when the data type of the field is the variable length character string, the confidence score 1% is set, when the data type of the field is real, the confidence score 5% is set, when the data type of the field is integer, the confidence score 80% is set, when the data type of the field is fixed length character string, the confidence score 70% is set, when the data type of the field is the time stamp or date, the confidence score 10% is set, and when the field name is designated as the main key, the confidence score 80% is set. Although items for the character string of the field value or field name are not defined here, some items for the character string may be defined. When the item for the field value is defined, it is referenced at the step S57.

For example, in case of the schema information as depicted in Fig. 4A, the confidence score 10% is identified for the field 1, because the data type is the time stamp, the confidence score 80%, which is further away form 50%, is adopted for the field 2, because the data type is the fixed length character string and the main key is assigned to the field 2, the confidence score 70% is identified for the field 3, because the data type is the fixed length character string, and the confidence score 1% is identified for the field 4, because the data type is the variable length character string. In a case where the schema information as depicted in Fig. 9A does not exist, the confidence score 50% is identified for the fields 1 to 4, because the pertinent item does not exist in the event ID and associated ID candidate confidence degree.

For example, in case of the schema information as depicted in Fig. 5A, because the data type is the time stamp, the confidence score 10% is set for the field 1. Because the data type is the fixed length character string and the main key is designated to the field 2, the confidence score 80%, which is further away from 50%, is adopted for the field 2. Because the data type is the fixed length character string, the confidence score 70% is identified for the fields 3 to 4, and because the data type is the time stamp, the confidence score 10% is set for the field 5. In case where the schema information as depicted in Fig. 10A does not exist, because any pertinent item does not exist in the event ID and associated ID candidate confidence score table, the confidence score 50% is identified for the fields 1 to 5.

For example, in case of the schema information as depicted in Fig. 6A, the confidence score 10% is identified for the field 1, because the data type is the time stamp, the confidence score 80%, which is further away from 50%, is identified for the field 2, because the data type is the fixed length character string and the main key is designated to the field 1, and the confidence score 70% is identified for the fields 3 to 5, because the data type is the fixed length character string. In case where the schema information as depicted in Fig. 11A does not exist, the confidence score 50% is identified for the fields 1 to 5, because, any pertinent items do not exist in the event ID and associated ID candidate confidence score table.

For example, in case of the schema information as depicted in Fig. 7A, the confidence score 10% is identified for the field 1, because the data type is the time stamp, the confidence score 80%, which is further away from 50%, is adopted for the field 2, because the data type is the fixed length character string and the main key is designated to the field 2, and the confidence score 70% is identified for the fields 3 to 4, because the data type is the fixed length character string. In the example that the schema information as depicted in Fig. 12A does not exist, the confidence score 50% is identified for the fields 1 to 4, because any pertinent items do not exist in the event ID and associated ID candidate confidence score table.

For example, in case of the schema information as depicted in Fig. 8A, the confidence score 80%, which is further away from 50%, is adopted for the field 1, because the data type is the fixed length character string and the main key is designated to the field 1, and the confidence score 70% is adopted for the field 2, because the data type is the fixed length character string. In the example that the schema information as depicted in Fig. 13A does not exit, the confidence score 50% is identified for the fields 1 and 2, because any pertinent items do not exist in the event ID and associated ID candidate confidence score table.

Then, the event ID and associated ID candidate processor 32 sets the confidence score identified at the step S57 to the event ID and associated ID candidate judgment, and stores the event ID and associated ID candidate judgment data into the storage device such as the main memory (step S59).

After that, the event ID and associated ID candidate processor 32 judges whether or not all fields have been processed in the processing target table (step S61), and when an unprocessed field exists, the processing returns to the step S51. On the other hand, when all of the fields have been processed, the processing returns to the calling source processing.

Thus, the greater confidence score is identified for the field whose probability that the field corresponds to the event ID or associated ID is high. In addition, "denial" is identified as the data representing probability that the field corresponds to the event ID or associated ID, when the probability that the field corresponds to the event ID or associated ID is completely zero.

Returning to the explanation of Fig. 3, next, the event name processor 34 of the event candidate data generator 3 carries out an event name judgment processing (step S13). This event name judgment processing will be explained by using Figs. 18 to 20.

First, the event name processor 34 counts the number of fields whose confidence score, which is the processing result of the time stamp judgment processing, is equal to or greater than a predetermined confidence score, and which can be considered to be the time stamp field (step S91). For example, 70% or more is set to a threshold as the predetermined confidence score. Naturally enough, the field for which "determined" is identified is the time stamp field. In the aforementioned example, except the product number DB, the number of fields is "1", because the field whose field name is the time is judged as the time stamp field. As for the product number DB, the number of fields is "0", because there is no field, which can be considered to be time stamp field.

Then, the event name processor 34 judges whether or not the number of fields for the time stamp is "0" (step S93). When the number of fields is "0", the event name processor 34 sets data representing the analysis target table is excluded from the tables to be analyzed in the following processing (step S95). The table having no time stamp (e.g. the product number table) is not judged as a table associated with the events, which occur during the business process. Then, the processing returns to the calling source processing.

On the other hand, when the number of fields of the time stamp is not "0", the event name processor 34 judges whether or not the number of fields is "1" (step S97). When the number of fields of the time stamp is "1", the event name processor 34 sets the table name to the event name, and stores the event name into the storage device such as the main memory (step S99). In the aforementioned example, in case of the order DB, "order" is identified as the event name, in case of the production DB, "production" is identified as the event name, in case of the arrangement DB, "arrangement" is identified as the event name, and in case of the delivery DB, "delivery" is identified. Then, the processing returns to the calling source processing.

In addition, when the number of fields of the time stamp is plural, the event name processor 34 sets the field name of the field, which is considered as the time stamp, to the event name, and stores the event name into the storage device such as the main memory (step S101). Then, the processing returns to the calling source processing.

For example, when the table as depicted in Fig. 19 is the processing target table, the step S101 is executed. In the example of Fig. 19, the fields "slip issuance time", "approval time", "order time", "delivery time", and "inspection time" are respectively considered as the field of the time stamp of the event, and a format that plural events are registered in one record is adopted. Such a table is considered as a table to which the slip issuance table, approval table, order table, delivery table and inspection table, which are depicted in Figs. 20A to 20E, are unified. Therefore, in such a case, "slip issuance", "approval", "order", "delivery" and "inspection" are respectively identified as the event names.

By carrying out the aforementioned processing, a table corresponding to an event, which occurs during the business process, can be identified, and the event names can also be extracted.

Returning to the explanation of Fig. 3, next, the event candidate data generator 3 presents the user with the judgment results through the input and output unit 11 (step S15). For example, in case of the order DB in the relational database format as depicted in Figs. 4A and 4B, data as depicted in Fig. 21 is presented to the user. In the example of Fig. 21, as for each of the time field, order number field, area field and order contents field, the judgment results at the steps S7 to S13 are presented. Incidentally, as for the event name, "denial" is indicated for all fields, because the table name is identified as the event name. According to this example, it is understood that, as for the time stamp field, "determined" is indicated for the time field, and the probability that the order number field or area field corresponds to the event ID or associated ID is high.

In addition, in case of the order DB in the CSV format as depicted in Fig. 9A, data as depicted in Fig. 22 is presented for the user. In the example of Fig. 22, the judgment results of the steps S7 to S13 are presented for each of the time field, order number field, area field and order contents field. Incidentally, as for the event name, "denial" is indicated for all fields, because the table name is identified as the event name. According to this presentation, the probability that the time field corresponds to the time stamp is high, and the probabilities that respective fields correspond to the event ID or associated ID are the same.

For example, in case of the production DB in the relational database format as depicted in Figs. 5A and 5B, data as depicted in Fig. 23 is presented to the user. In the example of Fig. 23, the judgment results of the step S7 to S13 are presented for each of the time field, production number field, order number field, product number field and due date field. Incidentally, as for the event name, "denial" is indicated for all fields, because the table name is identified as the event name. According to this example, it is understood that, as for the time stamp field, "determined" is indicated for the time field, and the probability that the production number field, order number field or product number field corresponds to the event ID or associated ID is high.

In addition, in the production DB in the CSV format as depicted in Fig. 10A, data as depicted it Fig. 24 is presented to the user. In the example of Fig. 24, the judgment results of the step S7 to S13 are presented for each of the time field, production number field, order number field, product number field and due date field. Incidentally, as for the event name, "denial" is indicated for all fields, because the table name is identified as the event name. According to this example, it is understood that the probability the time field corresponds to the time stamp is high, and the probability that the respective fields correspond to the event ID or associated ID is the same.

For example, in case of the arrangement DB in the relational database format as depicted in Figs. 6A and 6B, data as depicted in Fig. 25 is presented to the user. In the example of Fig. 25, the judgment results of the step S7 to S13 are presented for each of the time field, arrangement number field, order number field, product number field and delivery destination field. Incidentally, as for the event name, "denial" is indicated for all fields, because the table name is identified as the event name. According to this example, it is understood that, as for the time stamp field, "determined" is indicated for the time field, and the probability that the arrangement number field, order number field, product number field or delivery destination field corresponds to the event ID or associated ID is high.

For example, in case of the arrangement DB in the CSV format as depicted in Fig. 11A, data as depicted in Fig. 26 is presented to the user. In the example of Fig. 26, the judgment results of the step S7 to S13 are presented for each of the time field, arrangement number field, order number field, product number field and delivery destination field. Incidentally, as for the event name, "dental" is indicated for all fields, because the table name is identified as the event name. According to this example, it is understood that, as for the time stamp field, "determined" is indicated for the time field, and the probabilities that the respective fields correspond to the event ID or associated ID are equivalent.

For example, in case of the delivery DB in the relational database format as depicted in Figs. 7A and 7B, data as depicted in Fig. 27 is presented to the user. In the example of Fig. 27, the judgment results of the step S7 to S13 are presented for each of the time field, arrangement number field, delivery service field and delivery destination field. Incidentally, as for the event name, "denial" is indicated for all fields, because the table name is identified as the event name. According to this example, it is understood that, as for the time stamp field, "determined" is indicated for the time field, and the probability that the arrangement number field, delivery service field or delivery destination field corresponds to the event ID or associated ID is high.

For example, in case of the delivery DB in the CSV format as depicted in Fig. 12A, data as depicted in Fig. 28 is presented to the user. In the example of Fig. 28, the judgment results of the step S7 to S13 are presented for each of the time field, arrangement number field, delivery service field and delivery destination field. Incidentally, as for the event name, "denial" is indicated for all fields, because the table name is identified as the event name. According to this example, it is understood that, as for the time stamp field, "determined" is indicated for the time field, and the probabilities that the respective fields correspond to the event ID or associated ID are equivalent.

For example, in case of the product number DB in the relational database format as depicted in Figs. 8A and 8B, data as depicted in Fig. 29 is presented to the user. In the example of Fig. 29, the judgment results of the step S7 to S13 are presented for each of the product number field and product name field. Incidentally, as for the event name, "denial" is indicated for all fields, because it is judged that there is no time stamp and the product number DB is excluded from the tables to be analyzed in the following processing. According to this example, it is understood that the probability that the time stamp field exists is very low, and the probability that the product number field or product name field corresponds to the event ID or associated ID is high.

For example, in case of the product number DB in the CSV format as depicted in Fig. 13A, data as depicted in Fig. 30 is presented to the user. In the example of Fig. 30, the judgment results of the step S7 to S13 are presented for each of the product number field and product name field. Incidentally, as for the event name, "denial" is indicated for all fields, because it is judged that there is no time stamp and the product number DB is excluded from the tables to be analyzed in the following processing. According to this example, it is understood that the probability that the time stamp field exists is very low, and the probabilities that the respective fields correspond to the event ID or associated ID are equivalent.

Returning to the explanation of Fig. 3, when the step S15 is completed, the user conducts modification inputs or determination inputs for the event name, time stamp, the event ID and associated ID candidates and the like, conducts or instructs copy of records and the like, creates event candidate data, and stores the event candidate data into the event candidate data storage 5 (steep S16). Because this work is mainly or partially conducted by the user, the step S16 is indicated by a dotted line block in Fig. 3. Then, the processing returns to the step S3.

For example, according to the judgment results of Fig. 21, when the table name "order" is finally determined as the event name, the time field is finally determined as the time stamp, the order number field and the area field are finally determined as the event ID and associated ID candidates, data as depicted in Fig. 32 is stored into the event candidate data storage 5, for example. In the example of Fig. 32, the event name "order" is added to all of the records, the field values in the time field for all records are copied to the time stamp field, and the field names and field values in the order number field and area field for all records are copied as the event ID and associated ID candidates.

For example, according to the judgment results of Fig. 22, when the table name "order" is finally determined as the event name, the time field is finally determined as the time stamp, and the order number field, area field and order contents field are finally determined as the event ID and associated ID candidates, data as depicted in Fig. 33 is stored into the event candidate data storage 5, for example.

Furthermore, for example, according to the judgment results of Fig. 23, when the table name "production" is finally determined as the event name, the time field is finally determined as the time stamp, and the production number field and order number field are finally determined as the event ID and associated ID candidates, data as depicted in Fig. 34 is stored into the event candidate data storage 5, for example.

In addition, for example, according to the judgment results of Fig. 24, when the table name "production" is finally determined as the event name, the time field is finally determined as the time stamp, and the production number field and order number field are finally determined as the event ID and associated ID candidates, data as depicted in Fig. 35 is stored into the event candidate data storage 5, for example.

Furthermore, for example, according to the judgment results of Fig. 25, when the table name "arrangement" is finally determined as the event name, the time field is finally determined as the time stamp, and the arrangement number field and order number field are finally determined as the event ID and associated ID candidates, data as depicted in Fig. 36 is stored into the event candidate data storage 5, for example.

In addiction, for example, according to the judgment results of Fig. 26, when the table name "arrangement" is finally determined as the event name, the time field is finally determined as the time stamp, and the arrangement number field, order number field, product number field and delivery destination field are finally determined as the event ID and associated ID candidates, data as depicted in Fig. 37 is stored into the event candidate data storage 5, for example.

Moreover, for example, according to the judgment results of Fig. 27, when the table name "delivery" is finally determined as the event name, the time field is finally determined as the time stamp, and the arrangement number field, delivery service field and delivery destination field are finally determined as the event ID and associated ID candidates, data as depicted in Fig. 38 is stored into the event candidate data storage 5, for example.

In addition, for example, according to the judgment results of Fig. 28, when the table name "delivery" is finally determined as the event name, the time field is finally determined as the time stamp, and the arrangement number field, delivery service field and delivery destination field are finally determined as the event ID and associated ID candidates, data as depicted in Fig. 39 is stored into the event candidate data storage 5, for example.

In addition, when the table in which plural time stamp fields exist in one table as depicted, for example, in Fig. 19, is processed, data as depicted, for example, in Figs. 40 to 44 is stored in the event candidate data storage 5. In the examples of Figs. 40 to 44, based on the slip issuance time field, approval time field, order time field, delivery time filed and inspection time field, which are finally determined as the time stamp, the event candidate data in which the respective event names are finally determined as "slip issuance", "approval", "order", "delivery" and "inspection" is generated for each of those fields. As for the time stamp, the field values of the slip issuance time field, approval time field, order time field, delivery time field and inspection time field for all records are copied to the respective time stamp fields of the event candidate data. Furthermore, as for the fields other than the slip issuance time field, approval time field, order time field, delivery time field and inspection time field, the field names and field values for all records are respectively copied as the event ID and associated ID candidates to the event candidate data for each of those fields.

Thus, the event candidate data to be used in the following processing is stored into the event candidate storage 5.

When it is judged at the step S3 that all of the analysis target tables have been processed, the event data generator 7 carries out an event data generation processing by using the event candidate data stored in the event candidate data storage 5, and stores the processing result into the event data storage 9 (step S17).

An example of the event data is depicted in Fig. 45, which is generated by using one set of the event candidate data depicted in Figs. 32, 34, 36 and 38 or one set of the event candidate data as depicted in Figs. 33, 35, 37 and 39, respectively in association with the order event, production event, arrangement event and delivery event. As for the generation method of the event data, an automatic extraction method of the association information of the event data, which is described in the aforementioned Japanese Patent Application 2006-197294 may be used, or the association between the events may be finally determined by manually investigating and analyzing the correspondence relation of the field values of the event ID and associated ID candidates for the respective event candidate data.

In Fig. 45, it is finally determined that the event ID of the order event is the order number, the event ID of the production event is the production number, the associated ID is the order number, the event ID of the arrangement event is the arrangement number, the associated ID is the order number, and the event ID of the delivery event is the arrangement number, and the associated ID is the delivery service. In addition, the association between the events is finally determined, specifically, when it is identified that value of the field values of the event ID of the order event corresponds to a certain field value of the associated ID of the production event, a certain record (i.e. event instance) of the production event is associated with a specific record (i.e. event instance) of the order event. The similar associations between the association ID of the arrangement event and the event ID of the order event and between the event ID of the delivery event and the event ID of the arrangement event have been finally determined.

In addition, the process instance generator 13 carries out a process instance generation processing by using the event data stored in the event data storage 9, and stores the processing results into the process instance data storage 15 (step S19). A business process tracking method described in U.S. Patent Application Publication 2005/076059A1 or the like can be used as the generation method.

By using the event data of Fig. 45, outline explanation of a processing process to generate a process instance whose starting point is the order event instance of the order number: JT01 is depicted in Fig. 46A and 46B. First, as the records (i.e. event instance) whose field values of the associated ID is the field value: JT01 of the order number, which is the event ID of the order event, two event instances from the production event and three event instances from the arrangement event are determined. Next, as the records (i.e. event instance) whose field value of the associated ID is the arrangement number: TH01, TH02 or TH03, which was determined as the event ID of the arrangement event, three event instances from the delivery event are determined. Finally, by connecting the event instances having direct or indirect association from the determined order event instance of the order number: JT01 as the starting point in an order of the time progress based on the time stamp values, the process instance is generated. Namely, as the first process instance, a process instance in which event instances, "order", "production", "arrangement", "arrangement", "arrangement", "delivery", "production", "delivery" and "delivery" are arranged in time series, are generated.

Similarly, by using the event data in Fig. 45, all of the generated process instances are depicted in Fig. 47. The second process instance is a process instance that event instances "order", "arrangement" and "delivery" are arranged in time series. The third process instance is a process instance that event instances "order", "production", "production", "arrangement" and "delivery" are arranged in time series. Furthermore, the fourth process instance is a process instance that event instances "order", "arrangement" and "delivery" are arranged in time series.

Returning to the explanation of the processing flow in Fig. 3, next, the repetition canceller 17 carries out a repetition cancellation processing by using data of the process instances, which is stored in the process instance data storage 15 (step S21) . This processing will be explained in detail by using Figs. 48 to 62.

For a start, a purpose to carry out the repetition cancellation processing will be explained by using Figs. 48 to 52. First, as depicted in Fig. 48, it is assumed that 10 process instances are stored in the process instance data storage 15. Here, 5 process instances each including "Initial State", "contract", "slip preparation", "billing", "collection", "contract expiration" and "Final State" are generated and grouped as a group A. In addition, 3 process instances that, after "Initial State", "contract", "slip preparation", "billing" and "collection", a flow returns through "contract renewal" to "slip preparation", and after carrying out "billing" and "collection" (i.e. backtracking), the flow further shifts to "contract expiration" and "Final State" are generated and grouped as a group B. Furthermore, one process instance that, after "Initial State", "contract", "slip preparation", "billing" and "collection", a flow returns through "continuation" to "billing", and after "collection" is carried out (i.e. backtracked), the flow shifts to "contract expiration" and "Final State", is generated and grouped as a group C. Then, one process instance that, after "Initial State", "contract", "slip preparation", "billing" and "collection", "collection" is carried out again (i.e. repeated), and then a flow shifts to "contract expiration" and "Final State", is generated and grouped as a group D.

When such process instances are generated, and the process instances in the groups A to D are simply superimposed, an entire flow is generated as depicted in Fig. 49. In the entire flow of Fig. 49, the process instance in the group A is depicted by solid lines as a main flow, and passing event instance in the backtracking, backtracking transitions and repeated transitions, which are included in the groups B, C and D, are depicted by dotted lines in order to make it easy to see them for the convenience of the explanation.

In addition, for example, when by using, as a threshold, a ratio 20% of the appearance frequency of the group to the total number of process instances, the flows are categorized into the main flow and the exceptional flows, as depicted in Fig. 50A, a flow is generated, as the main flow, that the process instances in the groups A and B are superimposed, and shown for the user. On the other hand, as the exceptional flows, the process instance of the group C, which is depicted in Fig. 50B, (however, in order to make it easy to see the figure for the convenience of the explanation, the passing event instance and transition of the backtracking are depicted by the dotted lines.) and the process instance of the group D, which is depicted in Fig. 50C (however, in order to make it easy to see the figure for the convenience of the explanation, the transition representing the repetition is depicted by the dotted lines.) are shown for the user.

Thus, in case of the process instances depicted in Fig. 48, there is few problem on categorization of the process instances into the main flow and exceptional flows, and the user can easily understand the general situation of the business flows in the figure depicted in Figs. 49 and 50. Because the appearance frequency reaches 50% only by the group A, even if the group A is treated as the main flow, there is no special problem on understanding the general situation of the business flows, similarly to Fig. 50.

On the other hand, when the process instances as depicted in Fig. 51 is generated, this case is different from the case of Fig. 48, and a problem occurs. In the example of Fig. 51, a flow of "Initial State", "contact", "slip preparation", "bulling", "collection", "contract expiration" and "Final State" is used as a basic flow, and following process instances are generated, namely, two process instances in which the event instance "collection" is repeated once, one process instance in which the event instance "collection" is repeated twice, one process instance in which the event instance "collection" is repeated three times, one process instance in which the event instance "collection" is repeated four times, and one process instance in which the event instance "collection" is repeated five times. Also as for the remaining process instances, a flow of "Initial State", "contract", "slip preparation", "billing", "collection", "contract expiration" and "Final-State" is used as a basic flow, and following process instances are generated, namely, one process instance including a backtracking that one set of "slip reparation", "billing" and "collection" is repeated once through a passing event instance "contract ronewal", one process instance including a backtracking that one set of "slip preparation", "billing" and "collection" is repeated twice through the passing event instance "contract renewal", and one process instance including a backtracking that one set of "slip preparation", "billing" and "collection" is repeated three times through the passing event instance "contract renewal". Furthermore, a flow of "Initial State", "contract", "slip preparation", "billing", "collection", "contract expiration" and "Final State" is used as a basic flow, and one process instance including a backtracking that one set of "billing" and "collection" is repeated once through the passing event instance "continuation" is also generated.

Thus, when plural types of process instances that only the number of backtrackings is different and plural types of process instances that only the number of repetitions is different are generated and they are simply categorized, the number of process instances, which are judged as the same group, decreases very much. In the example of Fig. 51, process instances in which the event instance "collection" is repeated once are grouped because the number of process instances is "2". However, the appearance frequency is mere 20%. In addition, as depicted in Fig. 52, when other process instances are treated as the exceptional flows, 8 exceptional flows appear. Therefore, the meaning of the exceptional flows becomes vague on understanding the outline of the business flow.

Then, by carrying out a processing depicted in Figs. 53 to 62 to delete, from the process instances, the backtrackings and iterations that make it difficult to grasp the entire image of the business, it becomes possible to easily group the process instances, and for the user to easily grasp the outline of the business flow.

The repetition canceller 17 identifies one unprocessed process instance in the process instance data storage 15 (Fig. 52: step S111). Then, the repetition cancelled 17 examines whether or not the iteration exists and whether or not the backtracking exists in the identified process instance (step S113). A transition from a specific event instance to another event instance, which was carried out before the specific event instance, passing or without passing any passing event instance is identified as the backtracking, and a transition returning to the same event instance is identified as the iteration. One process instance may include the iteration, and backtracking, and furthermore, plural iterations or backtrackings may be included.

Then, the iteration processor 171 of the repetition canceller 17 judges whether or not all portions of the iterations in the identified process instance have been processed (step S115). When an unprocessed portion of the iterations exists, the iteration processor 171 identifies an unprocessed position of the iteration (step S117), leaves only one iteration at the identified position of the iteration and deletes other iterations (step S119). Then, the processing returns to the step S115.

For example, in case of the process instances as depicted in Fig. 54A, iterations 4001 occur at "slip preparation" three times, iterations 4002 occur at "billing" once, and iterations 4003 occurs at "billing start" four times. However, excess iterations are deleted for each position while only one iteration is left. Then, as depicted in Fig. 54B, one iteration 4001' is left at "slip preparation", one iteration 4002' is left at "billing" and one iteration 4003' is left at "billing start".

Returning to the explanation of the processing in Fig. 53, when all positions of the iterations have been processed or no iteration exists, the backtracking processor 173 judges whether or not all positions of the backtrackings have been processed (step S121). When an unprocessed position of the backtracking exists, the backtracking processor 173 identifies one unprocessed position of the backtracking (step S123). Then, the backtracking processor 173 carries out a backtracking repetition cancellation processing (step S125). The backtracking repetition cancellation processing will be explained by using Figs. 55 to 58B.

First, the backtracking processor 173 cuts out a backtracking portion at the identified position of the backtracking (step S131). Here, for example, a case where the process instance depicted in Fig. 56 is processed is assumed. Specifically, in this process instance, after the business proceeds to "Initial State", "contract", "slip preparation", "billing", "contract renewal" and "billing start", the business returns to "billing", and then after the business proceeds to "contract renewal" and "billing start", the business returns to "slip reparation", and after the business further proceeds to "billing" contract renewal" and "billing start", the business returns to "billing" and the business further proceeds to "contract renewal" and "billing start", and further to "billing end" and "Final State". At the step S131, as depicted in Fig. 57, a first backtracking portion to return to "billing", a second backtracking portion to return to "slip preparation" and a third backtracking portion to return to "billing" are out out.

Then, the backtracking processor 173 classifies patterns of the backtracking portions (step S133). As for three backtracking portions cut out as depicted in Fig. 58A, two backtracking portions to "billing", "contract renewal" and "billing start" are identified as pattern 1, and one backtracking portion to "slip preparation", "billing", "contact renewal" and "birling start" is identified as pattern 2.

Then, the backtracking processor 173 carries out a repetition cancellation for each pattern, namely, leaves one backtracking for each pattern and deletes the remaining backtrackings (step S135). When two patterns exist as depicted in Fig. 58A, the backtrackings are unified to one backtracking for each pattern as depicted in Fig. 58B.

After that, the backtracking processor 173 reconstructs the process instances, and stores the processing results into the simplified process instance data storage 19 (step S137). In case of Fig. 58B, as depicted in Fig. 59, by connecting the backtracking portions of the patterns 1 and 2 as event instances, which continuously occur, a process instance is constructed that event instances " Initial State", "contract", "slip preparation", "billing", "contract renewal", "billing start", "billing", "contract renewal", "billing start", "slip preparation", "contract renewal", "billing end" and "Final State" occur in this order.

When the process instances in the initial state depicted in Fig. 56 are displayed by superimposing the same event instances, complicated transitions are displayed as depicted in Fig. 60. However, by carrying out the aforementioned processing, as depicted in Fig. 61, it is clear that the backtrackings occur at two positions and it is possible to grasp the entire image.

Returning to the explanation of Fig. 53, the processing returns to the step S121 after the step S125.

When it is judged at the step S121 that all positions of the backtrackings have been processed, or no backtracking exists, the repetition canceller 17 judges whether or not all process instances have been processed (step S127). When an unprocessed process instance exists, the processing returns to the step S111. On the other hand, when no unprocessed process instance exists, the processing returns to the calling scarce processing.

Returning to the explanation of Fig. 3, the process instance classification processor 21 classifies the process instances stored in the simplified process instance data storage 19, counts the number of process instances for each type based on the classification result, and stores the counted value for each type into the model data storage 23 (step S23). When the process instances as depicted in Fig. 51 are generated and the step S21 is carried out, the process instances as depicted in Fig. 62 are stored into simplified process instance data storage 19. Namely, the process instances are classified into a group including 6 process instances each including the transition from "Initial State" through "contract", "slip preparation", "billing", "collection", "collection" and "contract expiration" to "Final State", into a group including 3 process instances each including the transition from "Initial State" through "contact", "slip preparation", "billing", "collection", "contract renewal", "slip preparation", "billing", "collection" and "contract expiration" to "Final tate", into a group including one process instance including the transition from "Initial State" through "contract", "slip preparation", "billing", "collection", "continuation", "billing", "collection" and "contract expiration" to "Final State". Therefore, data as depicted in Fig. 63 is stored into the model data storage 23. In the example of Fig. 63, the process instances of the aforementioned three groups and their counted values are registered. Incidentally, no data is registered at this stage into the column of the main flow flag.

Then, the process display processor 25 carries out a flow display processing by using data stored in the model data storage 23 (step S25). The flow display processing will be explained by using Figs. 64 to 66.

First, the flow display processor 25 arranges the groups of the process instances stored in the model data storage 23 based on the counted values in descending order (step S141). Then, the flow display processor 25 determines a threshold for a ratio of the number of process instances in the group to the total number of process instances, by an input value when the user inputs or a preset value when the user does not input (step S143). The threshold is a judgment reference to judge whether or not the group of each process should be treated as a main flow. For example, 20% is inputted, when the group whose ratio to the total number of process instances is equal to or greater than 20% is categorized into the main flow. However, the present value (e.g. 30%) itself may be used.

Then, the flow display processor 25 selects one unselected process instance in descending order of the counted value (step S147). The process display processor 25 designates this selected process instance as the main flow (also called typical flow) (step S149). Specifically, the process display processor 25 sets ON to a main flow flag in the table of the model data storage 23. Then, the process display processor 25 judges whether or not the ratio of the respective groups to the total number is equal to or greater than the threshold (step S153). When this condition is satisfied, the processing returns to the step S147.

For example, in the example of Fig. 63, first, when the first record is selected, the ratio to the total is equal to 60%, and the processing returns to the step S147 when the threshold is equal to 20%. Next, when the second record is selected, the ratio to the total is equal to 30%, and similarly the processing returns to the step S147. Thus, ON is set to the main flow flags for the first and second records.

Finally, when the third record is selected, the ratio to the total is equal to 10%, and the condition that the ratio to the total is equal to or greater than the threshold is not satisfied. Therefore, the flow display processor 25 makes the processing return to the calling source. By doing so, because ON is not set to the main flow flag for the process instances belonging to the groups other than the groups of the process instances selected at the step S147, they are identified as the exceptional flow.

Returning to the explanation of Fig. 3, the flow display processor 25 outputs the processing result through the input and output unit 11 by using data stored in the model data storage 23 (step S27). For example, when superimposing and displaying all of the process instances, the business flow as depicted in Fig. 65 is displayed. As depicted in Fig. 65, a flow including only one backtracking passing through "continuation", one backtracking passing through "contract renewal" and one iteration of "collection" is displayed.

In addition, when the main flows and exceptional flows are display separately by using data of the main flow flags stored in the model data storage 23, display as depicted in Fig. 66 is made. For example, when 90% is adopted as the classification ratio, the process instances for the first and second records in the table depicted in Fig. 63 are superimposed, and a business flow depicted in an upper stage of Fig. 66 is displayed as the main flow. In addition, the third process instance in the table depicted in Fig. 63 is displayed as the exceptional flow.

When such a processing is carried out, because the business flow is shown in a finely arranged form compared with the classification and display depicted in Fig. 52, it becomes possible for the user to grasp the business flow, which is actually carried out. Namely, because the backtrackings and iterations, which make it difficult to understand the entire image of the business) on grasping the feature, are omitted, the presence and/or manner of the iterations and the presence and/or manner of the backtrackings are easily grasped.

Although one embodiment of this invention was explained above, this invention is not limited to this embodiment. For example, the functional block diagram depicted in Fig. 1 is a mere example, and the diagram does not always correspond to actual program modules.

In addition, in case of reconstructing the process instances by deleting the backtrackings, which make it difficult to grasp the entire image of the business, when plural backtrackings occurs at the same position as depicted in Fig. 59, they are recognized as difference process instances unless a certain rule for the order is defined. For example, when a rule is adopted that the process instances are arranged in descending order of the length of the backtracking and reconstructed, a case disappears where the substantially same process instances whose backtracking order is different are generated.

In addition, the respective score tables are mere examples, and a method for setting the confidence score values may be determined more specifically, based on the experiences. Furthermore, as for the items of the score table, the number of items may be lesser or greater.

In addition, in the processing flow of Fig. 3, the order of the steps S7 to S13 may be rearranged, and the steps S7 to S13 may be executed in parallel.

In addition, at the output of the judgment result, the fields, which is definitely judged in each judgment item, and whose confidence score is equal to or greater than a predetermined threshold may be automatically selected and shown to the user, and then any selection or input may be prompted to the user for the judgment item, which cannot be automatically selected.

Furthermore, a processing loop for the processing target field is included in each of the steps S7 to S13. However, the processing loop for the processing target field may be taken outside the steps S7 to S13.

Incidentally, the business system analysis apparatus is a computer device as shown in Fig. 67. That is, a memory 2501 (storage device), a CPU 2503 (processor), a hard disk drive (HDD) 2505, a display controller 2507 connected to a display device 2509, a drive device 2513 for a removal disk 2511, an input device 2515, and a communication controller 2517 for connection with a network are connected through a bus 2519 as shown in Fig. 67. An operating system (OS) and an application program for carrying out the foregoing processing in the embodiment, are stored in the HDD 2505, and when executed by the CPU 2503, they are read out from the HDD 2505 to the memory 2501. As the need arises, the CPU 2503 controls the display controller 2507, the communication controller 2517, and the drive device 2513, and causes them to perform necessary operations. Besides, intermediate processing data is stored in the memory 2501, and if necessary, it is stored in the HDD 2505. In this embodiment of this invention, the application program to realize the aforementioned functions is stored in the computer-readable removal disk 2511 and distributed, and then it is installed into the HDD 2505 from the drive device 2513. It may be installed into the HDD 2505 via the network such as the Internet and the communication controller 2517. In the computer as stated above, the hardware such as the CPU 2503 and the memory 2501, the OS and the necessary application program are systematically cooperated with each other, so that various functions as described above in detail are realized.

## Claims

1. A business flow processing program for causing a computer to execute a procedure comprising:
extracting data of a series of works carried out for each case from a database storing results of said works, generating and storing into a process instance data storage device, process instances in which work names of said works carried out are arranged in time series;
judging, for each of said process instances stored in said process instance data storage device, whether or not a backtracking from a first work to a second work carried out prior to said first work occurs;
deleting, for each type of backtracking patterns, an additional backtracking from said process instance in which said backtracking occurs, and storing a process instance after deletion of said additional backtracking into a simplified process instance data storage device;
counting, for each process type, a number of process instances in said simplified process instance data storage device; and
identifying, based on counting results, process instances whose appearance frequency is equal to or greater than a predetermined reference and are stored in said simplified process instance data storage device, and outputting the identified process instance as a main business flow.

2. The business flow processing program as set forth in claim 1, wherein said procedure further comprises:
judging, for each said process instance stored in said process instance data storage device, whether or not an iteration from a third work in said process instance to said third work occurs; and
deleting, for each type of iteration patterns, an additional iteration from said process instance that said iteration occurs, and storing said process instance after the deletion of said additional iteration into said process instance data storage device.

3. The business flow processing program as set forth in claim 1, wherein said procedure further comprises:
judging, for each said process instance stored in said process instance data storage device, whether or not an iteration from a third work in said process instance to said third work occurs; and
deleting, for each type of iteration patterns, an additional iteration from said process instance that said iteration occurs, and storing said process instance after the deletion of said additional iteration into said simplified process instance data storage device.

4. The business flow processing program as set forth in claim 1, wherein said outputting comprises superimposing the identified process instances.

5. The business flow processing program as set fort in claim 1, wherein said outputting comprises outputting, as exceptional flows, said process instances other than the identified process instances.

6. A business flow processing method, comprising:
extracting data of a series of works carried out for each case from a database storing results of said works, generating and storing into a process instance data storage device, process instances in which work names of said works carried out are arranged in time series;
judging, for each of said process instances stored in said process instance data storage device, whether or not a backtracking from a first work to a second work carried out prior to said first work occurs;
deleting, for each type of backtracking patterns, an additional backtracking from said process instance in which said backtracking occurs, and storing a process instance after deletion of said additional backtracking into a simplified process instance data storage device;
counting, for each process type, a number of process instances in said simplified process instance data storage device; and
identifying, based on counting results, process instances whose appearance frequency is equal to or greater than a predetermined reference and are stored in said simplified process instance data storage device, and outputting the identified process instance as a main business flow.

7. A business flow processing apparatus, comprising:
means for extracting data of a series of works carried out for each case from a database storing results of said works, generating and storing into a process instance data storage device, process instances in which work names of said works carried out are arranged in time series;
means for judging, for each of said process instances stored in said process instance data storage device, whether or not a backtracking from a first work to a second work carried out prior to said first work a occurs;
means for deleting, for each type of backtracking patterns, an additional backtracking from said process instance in which said backtracking occurs, and storing a process instance after deletion of said additional backtracking into a simplified process instance data storage device;
means for counting, for each process type, a number of process instances in said simplified process instance data storage device; and
means for identifying, based on counting results, process instances whose appearance frequency is equal to or greater than a predetermined reference and are stored in said simplified process instance data storage device, and outputting the identified process instance as a main business flow.
